# EUROPEAN PATENT APPLICATION

(11) **EP 4 480 405 A1**
(43) Date of publication of application: **25.12.2024**
(21) Application number: 23756445.5
(22) Date of filing: 16.02.2023
(51) Int. Cl.: A61B 5/055, G01N 33/66, G01N 33/68, G06T 7/00, G06T 7/60, G06V 10/80

(54) **SUPPORT FOR DIAGNOSIS OF NEUROPSYCHIATRIC SLE BY MEANS OF HEAD MRI**

(30) Priority: 17.02.2022 JP 2022023098
(71) Applicant: NAGASAKI UNIVERSITY, Nagasaki 852-8521 (JP)
(72) Inventor: URASHIMA, Kayoko, Nagasaki-shi, Nagasaki 852-8521 (JP); ICHINOSE, Kunihiro, Nagasaki-shi, Nagasaki 852-8521 (JP); ISHIMARU, Hideki, Nagasaki-shi, Nagasaki 852-8521 (JP); UEKI, Masao, Nagasaki-shi, Nagasaki 852-8521 (JP)
(74) Representative: J A Kemp LLP
(86) International application number: PCT/JP2023/005474
(87) International publication number: WO 2023/157920

(57) **Abstract**

The present invention provides a method for obtaining an indicator for diagnosing whether a subject is an SLE patient or an NPSLE patient. The method includes: (1) preparing head MRI images of SLE patients and NPSLE patients; (2) gradually changing a color tone of each of the head MRI images; (3) detecting and counting white connected components and black connected components generated in response to color tone changes; (4) obtaining a scatter diagram of times of generation and disappearance of the white connected components and black connected components based on the a result of the detecting and counting (3); (5) verifying whether there is a significant difference in any of image features between SLE patients and NPSLE patients, the image features being obtained from the scatter diagram; and (6) identifying, as the indicator, an image feature in which a significant difference is recognized.

## Description

### Technical Field

The present invention relates to a method for obtaining an indicator for assisting diagnosis of neuropsychiatric SLE by magnetic resonance imaging (MRI) of the head, a method for assisting diagnosis of neuropsychiatric SLE using the indicator, and the like.

### Background Art

Neuropsychiatric SLE (NPSLE), which is a neurological symptom of systemic lupus erythematosus (SLE), presents non-specific and diverse symptoms such as peripheral neuropathies, headaches, and depressions. The NPSLE involves white-matter lesions, atrophy, and the like observed in head MRI images, but these signs are not specific. At the time of diagnosis, in addition to head MRI, a blood test and a cerebrospinal fluid test are performed. However, no tests involve a clear biomarker for diagnostic confirmation, and thus the diagnostic confirmation largely depends on the judgment of physicians, and diagnostic accuracy varies.

A recent study reports that the neuropsychiatric SLE has been found to have a cerebral blood flow different from that in a healthy person (NPL 1). However, normal medical care does not include fMRI or testing of the amount of cerebral blood perfusion, and thus applying the recent finding to normal medical care is difficult. Although white-matter lesions generally observed in NPSLE patients are not specific to NPSLE, there is a report that NPSLE patients with neurologic symptoms have been found in head MRI to have significantly more lesions with a size of at least 10 mm than SLE patients without psychiatric symptoms (NPL 2), but this report has not yet sufficiently assisted the physician in diagnosing a subject as having NPSLE.

### Citation List

### Non-Patent Literature

NPL 1: Papadaki et al., Ann Rheum Dis. 2018; 77: 441-449
NPL 2: Katsumata et al., BMC Musculoskeletal Disorders 2010, 11:13

### Summary of Invention

### Technical Problem

Therefore, an object of the present invention is to provide, for neuropsychiatric SLE being difficult for image diagnosis so far, an imaging biomarker for diagnostic imaging of NPSLE, which may sufficiently assist a physician in definitive diagnosis of neuropsychiatric SLE by comparing image data of head MRI between NPSLE patients and SLE patients using a data science technique, extracting a parameter for diagnosing the patient as having NPSLE, and evaluating the determination accuracy for NPSLE based on the parameter. Another object of the present invention is to provide a method for assisting diagnosis of NPSLE using the imaging biomarker. Yet another object of the present invention is to provide a therapeutic method and the like in which an appropriate therapeutic agent is administered after determining whether the patient has NPSLE or SLE using the imaging biomarker.

### Solution to Problem

In order to obtain a novel imaging biomarker for discriminating NPSLE from SLE, the inventors contemplated that, in consideration of the contents of the above-mentioned documents in related arts and the like, there is a need to change the viewpoint of using, as an indicator for head MRI images, a white-matter lesion or the like that is visible at a glance. Thus, the inventors focused on a technique called topological data analysis, and conceived of analyzing head MRI images (which may hereinafter be referred to as "brain images") of the patient by this technique. In the topological data analysis, the inventors paid attention to a technique called persistent homology, and for so-called "holes" that are connected components generated when this technique is used, created a scatter diagram (persistence diagram) based on generation and disappearance of the "holes". As a result of intensive studies, the inventors have found that the value of the perimeter of a convex peel containing at least 95% of the plots in the scatter diagram is useful as an indicator (imaging biomarker) for discriminating NPSLE from SLE. In addition, as a result of further studies, the inventors have found that the area value of a convex peel containing at least 95% of the plots in the scatter diagram is also useful as an indicator (imaging biomarker) for discriminating NPSLE from SLE. As a result of further studies based on these findings, the inventors have completed the present invention.

Specifically, the present invention is as follows.
[1] A method for obtaining an indicator for diagnosing whether a subject is an SLE patient or an NPSLE patient, the method including:
   (1) preparing head MRI images of SLE patients and NPSLE patients;
   (2) gradually changing a color tone of each of the head MRI images;
   (3) detecting and counting white connected components and black connected components generated in response to color tone changes;
   (4) obtaining a scatter diagram of times of generation and disappearance of the white connected components and the black connected components based on a result of the detecting and counting (3);
   (5) verifying whether there is a significant difference in any of image features between the SLE patients and the NPSLE patients, the image features being obtained from the scatter diagram; and
   (6) identifying, as the indicator, an image feature in which a significant difference is recognized.
[2] The method according to [1], in which
   the preparing (1) includes pre-processing the head MRI images.
[3-1] The method according to [1] or [2], in which
   in the verifying (5), the image features are obtained based on a convex peel containing at least 90% of plots in the scatter diagram.
[3-2] The method according to [1] or [2], in which
   in the verifying (5), the image features are obtained based on a convex peel containing at least 95% of plots in the scatter diagram.
[4-1] The method according to [3-1] or [3-2], in which the image features are obtained based on a length of a perimeter of the convex peel containing at least 90% of the plots in the scatter diagram.
[4-2] The method according to [3-1] or [3-2], in which the image features are obtained based on a length of a perimeter of the convex peel containing at least 95% of the plots in the scatter diagram.
[5] A method for assisting diagnosis of whether a subject is an SLE patient or an NPSLE patient, the method including:
   (1) preparing a head MRI image of a subject;
   (2) gradually changing a color tone of the head MRI image;
   (3) detecting and counting white connected components or black connected components generated in response to color tone changes;
   (4) obtaining a scatter diagram of times of generation and disappearance of the white connected components and the black connected components based on a result of the detecting and counting (3);
   (5) calculating a specific image feature from the scatter diagram; and
   (6) determining whether the subject is an SLE patient or an NPSLE patient from the calculated image feature.
[6] The method according to [5], in which
   the preparing (1) includes pre-processing the head MRI image.
[7-1] The method according to [5] or [6], in which
   in the calculating (5), the specific image feature is a length of a perimeter of a convex peel containing at least 90% of plots in the scatter diagram.
[7-2] The method according to [5] or [6], in which
   in the calculating (5), the specific image feature is a length of a perimeter of a convex peel containing at least 95% of plots in the scatter diagram.
[8] The method according to [7-1] or [7-2], in which
   in the determining (6), whether the subject is an SLE patient or an NPSLE patient is determined by comparing the length of the perimeter calculated in the calculating (5) with a predetermined cutoff value.
[9-1] The method according to [5] or [6], in which
   in the calculating (5), the specific image feature is an area of a convex peel containing at least 90% of plots in the scatter diagram.
[9-2] The method according to [5] or [6], in which
   in the calculating (5), the specific image feature is an area of a convex peel containing at least 95% of plots in the scatter diagram.
[10] The method according to [9-1] or [9-2], in which
   in the determining (6), whether the subject is an SLE patient or an NPSLE patient is determined by comparing the area calculated in the calculating (5) with a predetermined cutoff value.
[11] The method according to any one of [5] to [7-2], [9-1], and [9-2], in which
   in the determining (6), whether the subject is an SLE patient or an NPSLE patient is determined from:
   the calculated image feature; and
   one or more indicators selected from the group consisting of an age of the subject, an amount of prednisolone orally administered, an amount of C3 in serum, a blood glucose level, and an amount of IL-6 in the cerebrospinal fluid.
[12] The method according to [11], in which
   the indicator includes an age of the subject.
[13] A computer program for predicting whether a subject is an SLE patient or an NPSLE patient, the computer program causing a computer to function as:
   a reception unit (P1) configured to receive head MRI image data of a prediction target;
   a calculation unit (P2) configured to
      (i) gradually change a color tone of the received head MRI image data,
      (ii) detect and count white connected components or black connected components generated in response to color tone changes,
      (iii) obtain a scatter diagram of times of generation and disappearance of the white connected components and the black connected components based on a result of the detecting and counting,
      (iv) calculate a specific image feature from the scatter diagram, and
      (v) calculate at least one of a probability that the subject is an SLE patient or a probability that the subject is an NPSLE patient; and
   an output unit (P3) configured to output a calculation result obtained by the calculation unit.
[13-1] The program according to [13], in which
   in the calculation unit (P2), the specific image feature is a length of a perimeter of a convex peel containing at least 90% of plots in the scatter diagram.
[13-2] The program according to [13], in which
   in the calculation unit (P2), the specific image feature is a length of a perimeter of a convex peel containing at least 95% of plots in the scatter diagram.
[13-3] The program according to [13], in which
   in the calculation unit (P2), the specific image feature is an area of a convex peel containing at least 90% of plots in the scatter diagram.
[13-4] The program according to [13], in which
   in the calculation unit (P2), the specific image feature is an area of a convex peel containing at least 95% of plots in the scatter diagram.
[14] A method for treating or preventing NPSLE or SLE, the method including:
   (1) preparing a head MRI image of a subject;
   (2) gradually changing a color tone of the head MRI image;
   (3) detecting and counting white connected components or black connected components generated in response to color tone changes;
   (4) obtaining a scatter diagram of times of generation and disappearance of the white connected components and the black connected components based on a result of the detecting and counting (3);
   (5) calculating a specific image feature from the scatter diagram;
   (6) determining whether the subject is an SLE patient or a NPSLE patient from the calculated image feature; and
   (7) administering a therapeutic agent for NPSLE or SLE to the subject determined to have NPSLE or SLE based on a determination result in the determining (6).
[14-1] The method according to [14], in which
   in the calculating (5), the specific image feature is a length of a perimeter of a convex peel containing at least 90% of plots in the scatter diagram.
[14-2] The method according to [14], in which
   in the calculating (5), the specific image feature is a length of a perimeter of a convex peel containing at least 95% of plots in the scatter diagram.
[14-3] The method according to [14], in which
   in the calculating (5), the specific image feature is an area of a convex peel containing at least 90% of plots in the scatter diagram.
[14-4] The method according to [14], in which
   in the calculating (5), the specific image feature is an area of a convex peel containing at least 95% of plots in the scatter diagram.

### Advantageous Effects of Invention

According to the present invention, an imaging biomarker (indicator) for diagnosing whether a subject is an SLE patient or an NPSLE patient (NPSLE positive or negative) can be obtained. In addition, using the imaging biomarker obtained by the present invention allows an NPSLE group to be distinguished from an SLE group with NPSLE non-progressing based on head MRI images. Furthermore, the present invention does not require the introduction of special equipment, and thus the method can be applied to normal medical care. Furthermore, since the present invention can distinguish between the NPSLE group and the SLE group with NPSLE non-progressing, the present invention can assist a physician in a definitive diagnosis, and the result of the distinction can assist appropriate drug intervention for NPSLE or SLE.

### Brief Description of Drawings

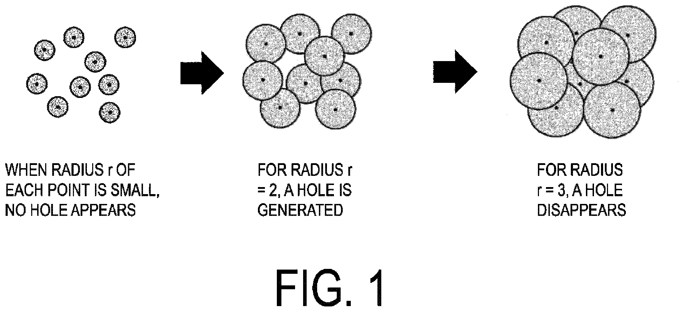

FIG. 1 is a diagram illustrating an image (generation and disappearance of holes when a radius r is changed) in which filtration is constructed.

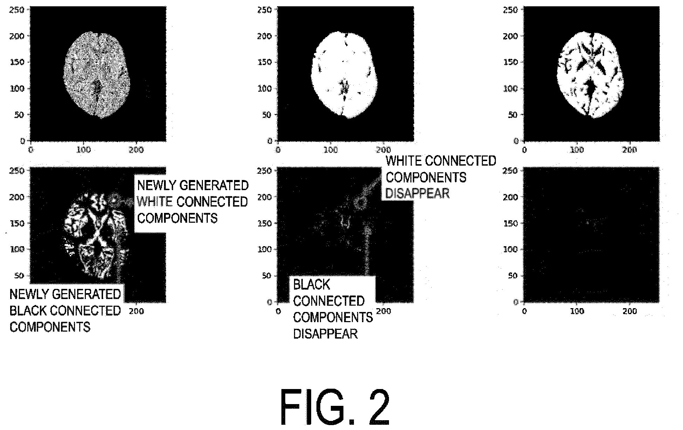

FIG. 2 is a diagram illustrating generation and disappearance of white connected components and black connected components in response to gradual changes in a threshold value for a color tone of an MRI image.

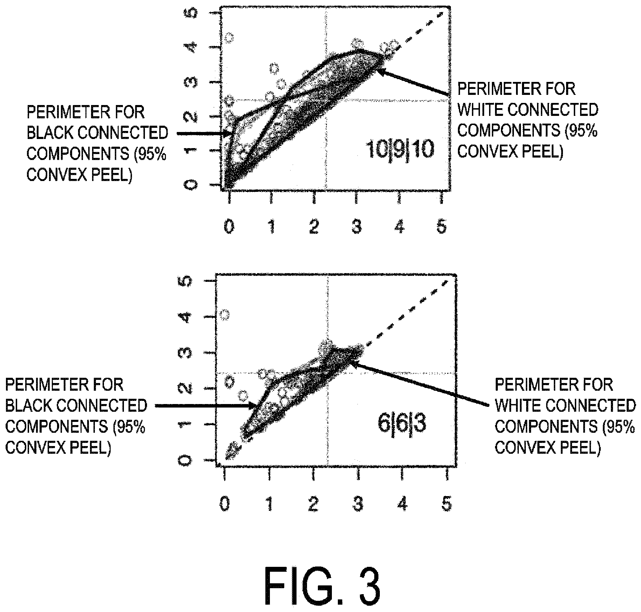

FIG. 3 is a diagram for describing a perimeter of a 95% convex peel obtained from a scatter diagram created based on the generation and disappearance of white connected components and black connected components.

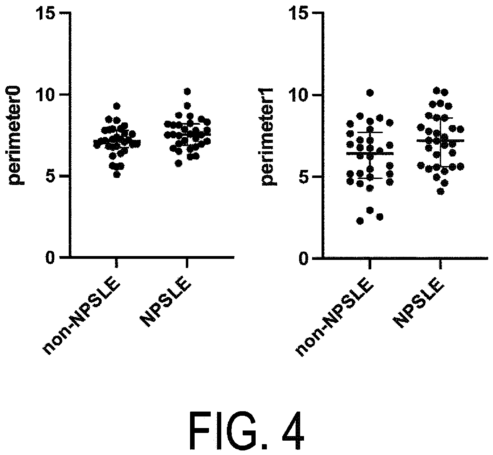

FIG. 4 is a scatter diagram of the perimeter (perimeter0) of black connected components and the perimeter (perimeter1) of white connected components.

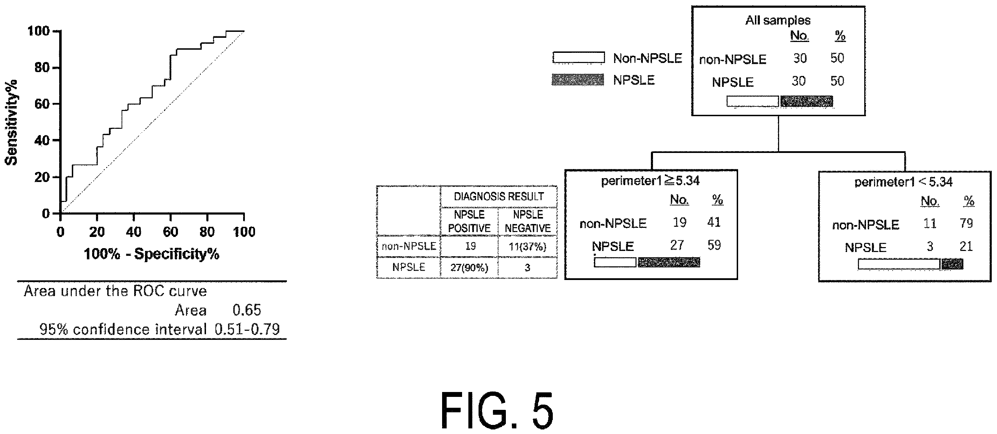

FIG. 5 left is a diagram illustrating an ROC curve of the perimeter (perimeter1) of the white connected components, and an AUC and a 95% confidence interval. FIG. 5 right is a diagram illustrating discrimination of NPSLE based on the perimeter (perimeter1) of the white connected components using a decision tree method.

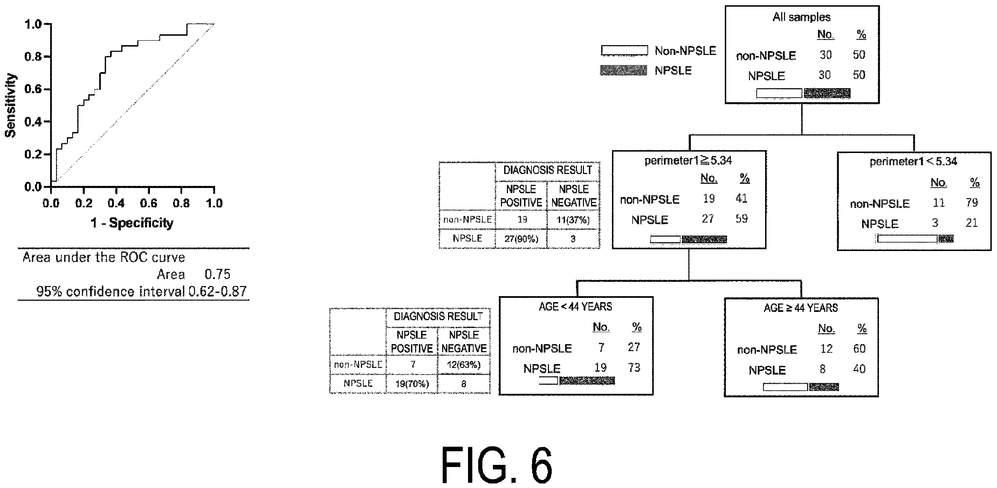

FIG. 6 left is a diagram illustrating an ROC curve of the age + the perimeter (perimeter1) of the white connected components, and the AUC and the 95% confidence interval. FIG. 6 right is a diagram illustrating discrimination of NPSLE based on the age + the perimeter (perimeter1) of the white connected components using the decision tree method.

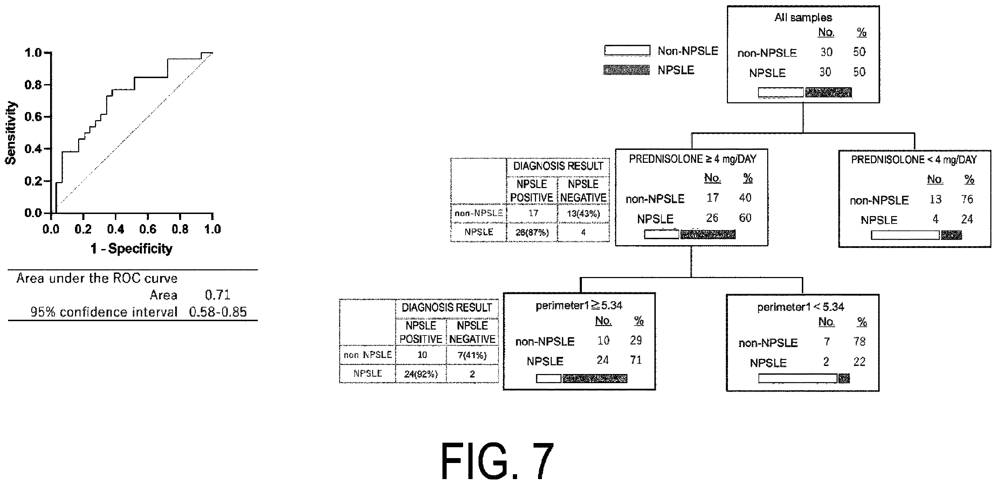

FIG. 7 left is a diagram illustrating an ROC curve of the amount of prednisolone orally administered + the perimeter (perimeter1) of the white connected components, and the AUC and the 95% confidence interval. FIG. 7 right is a diagram illustrating discrimination of NPSLE based on the amount of prednisolone orally administered + the perimeter (perimeter1) of the white connected components using the decision tree method.

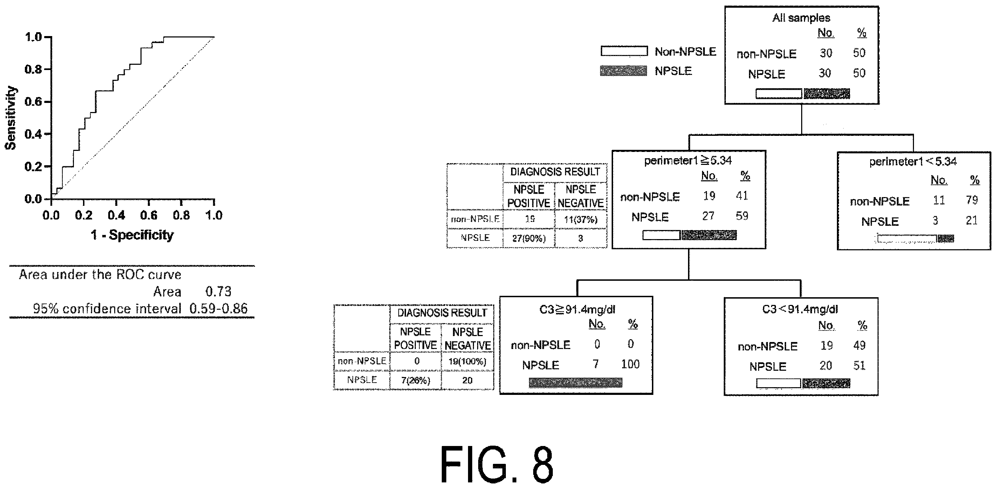

FIG. 8 left is a diagram illustrating an ROC curve of C3 in serum + the perimeter (perimeter1) of the white connected components, and the AUC and the 95% confidence interval. FIG. 8 right is a diagram illustrating discrimination of NPSLE based on C3 in serum + the perimeter (perimeter1) of the white connected components using the decision tree method.

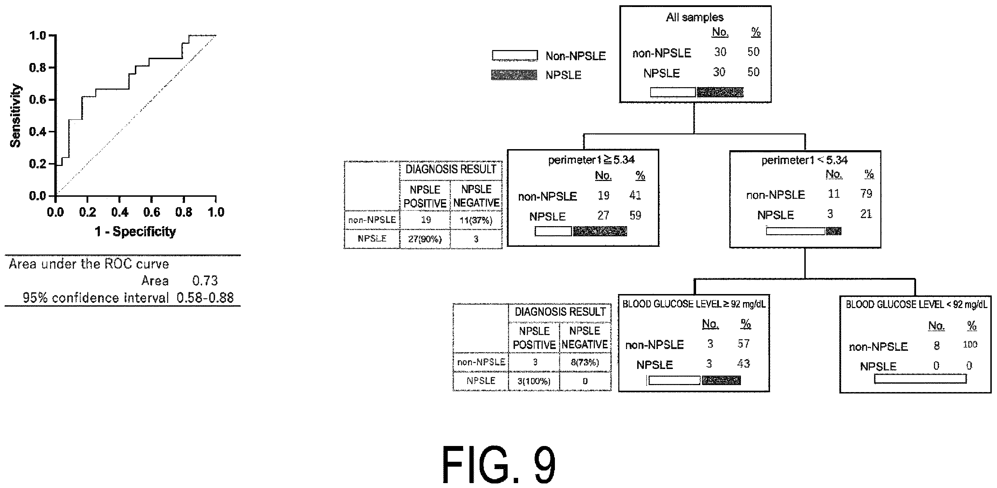

FIG. 9 left is a diagram illustrating an ROC curve of the blood glucose level + the perimeter (perimeter1) of the white connected components, the AUC and the 95% confidence interval. FIG. 9 right is a diagram illustrating discrimination of NPSLE based on the blood glucose level + the perimeter (perimeter1) of the white connected components using the decision tree method.

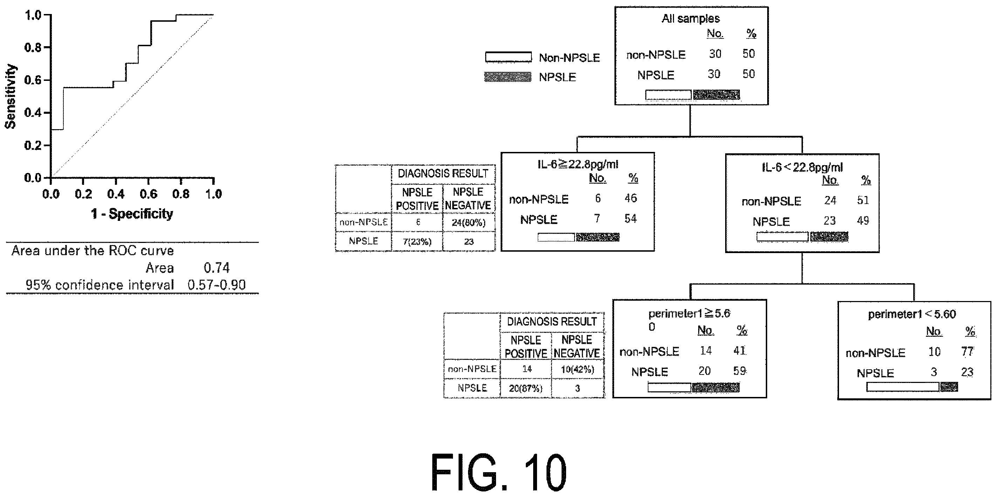

FIG. 10 left is a diagram illustrating an ROC curve of IL-6 in the cerebrospinal fluid + the perimeter (perimeter1) of the white connected components, and the AUC and the 95% confidence interval. FIG. 10 right is a diagram illustrating discrimination of NPSLE based on IL-6 in the cerebrospinal fluid + the perimeter (perimeter1) of the white connected components using the decision tree method.

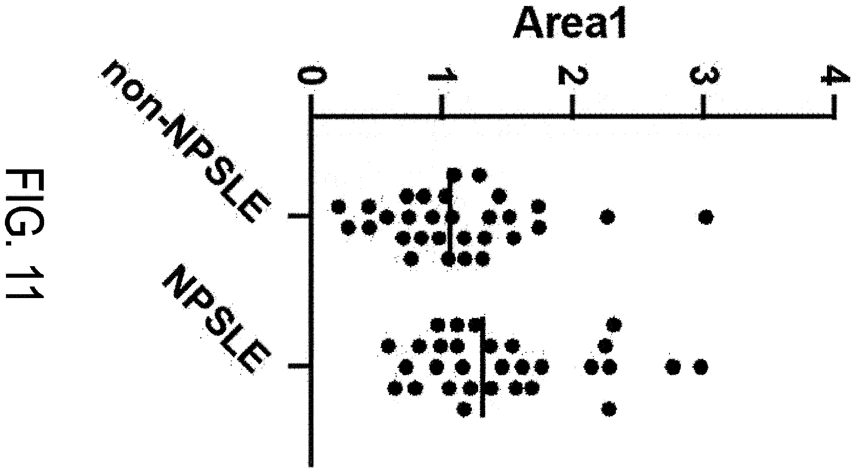

FIG. 11 is a scatter diagram of the area (area1) of the white connected components.

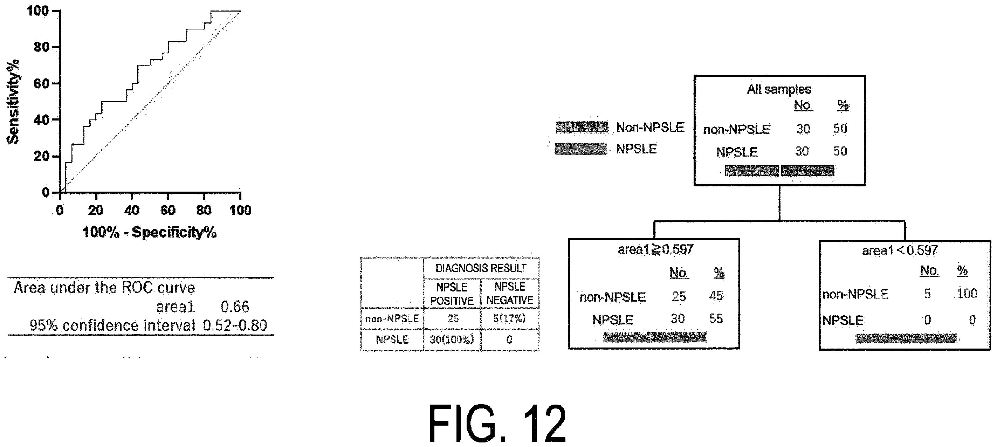

FIG. 12 left is a diagram illustrating an ROC curve of the area (area1) of the white connected components, and the AUC and the 95% confidence interval. FIG. 12 right is a diagram illustrating discrimination of NPSLE based on the area (area1) of the white connected components using the decision tree method.

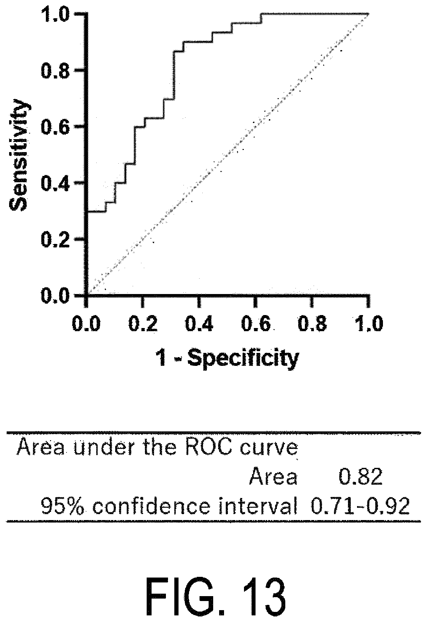

FIG. 13 is a diagram illustrating an ROC curve of the age + the history of cerebrovascular disorder + the serum complement value (CH50) + the area (area1) of the white connected components, and the AUC and the 95% confidence interval.

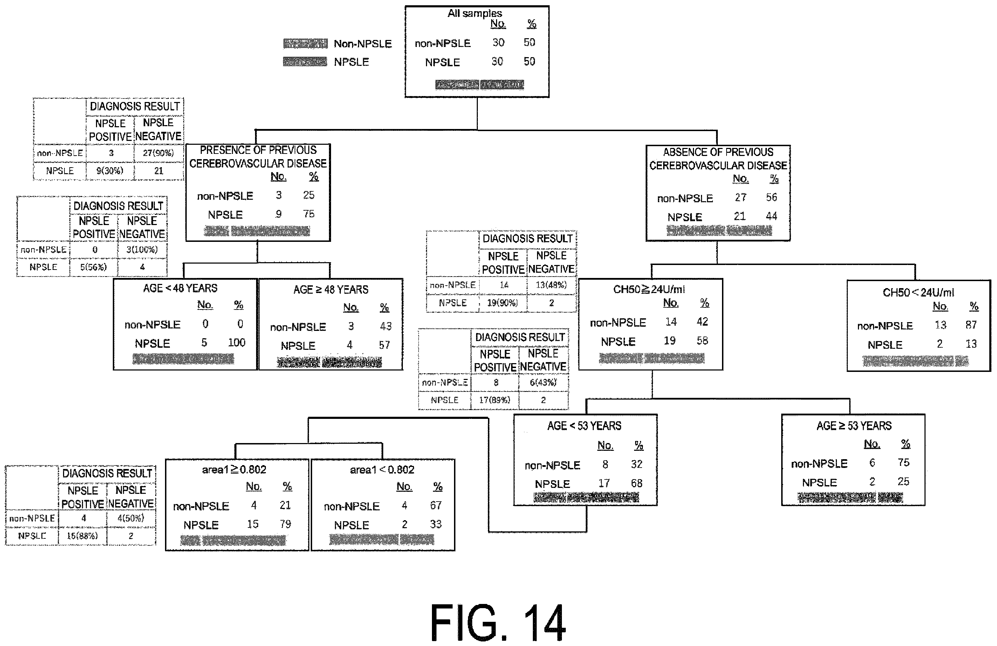

FIG. 14 is a diagram illustrating discrimination of NPSLE based on the age + the history of cerebrovascular disorder + the serum complement value (CH50) + the area (area1) of the white connected components using the decision tree method.

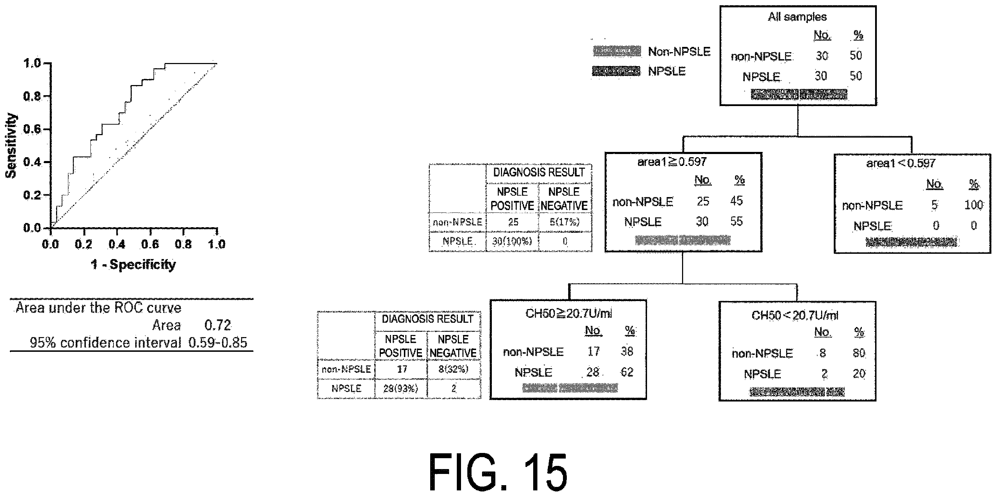

FIG. 15 left is a diagram illustrating an ROC curve of the serum complement value (CH50) + the area of the white connected components (area1), and the AUC and the 95% confidence intervals. FIG. 15 right is a diagram illustrating discrimination of NPSLE based on the serum complement value (CH50) + the area of the white connected components (area1) using the decision tree method.

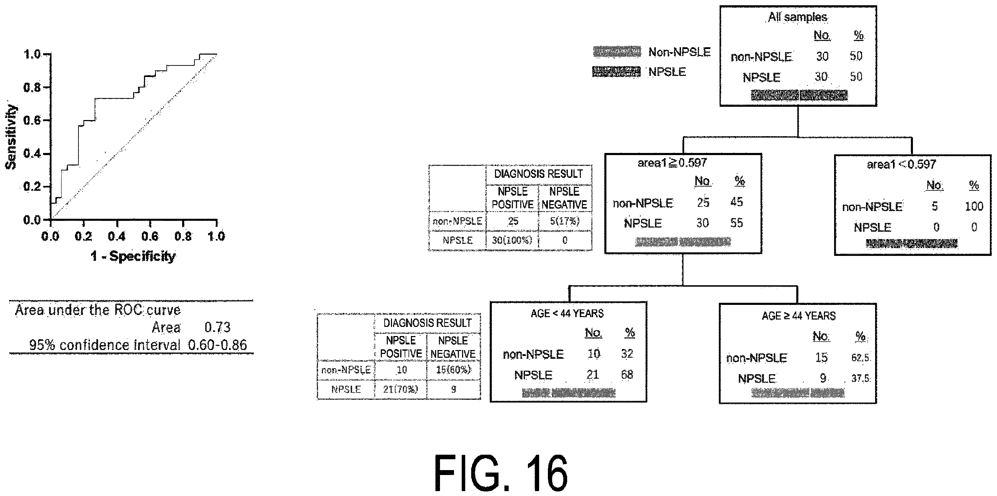

FIG. 16 left is a diagram illustrating an ROC curve of the age + the area (area1) of the white connected components, and the AUC and the 95% confidence interval. FIG. 16 right is a diagram illustrating discrimination of NPSLE based on the age + the area (area1) of the white connected components using the decision tree method.

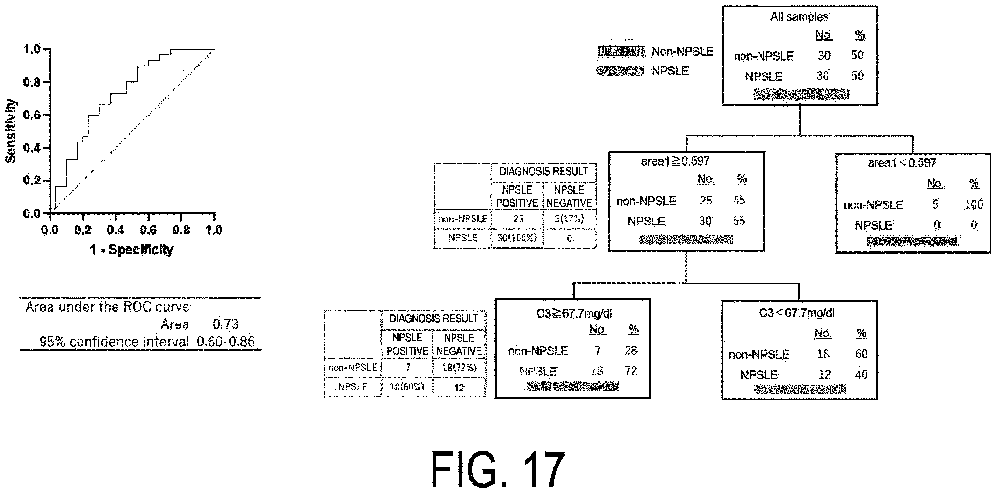

FIG. 17 left is a diagram illustrating an ROC curve of C3 in serum + the area (area1) of the white connected components, and the AUC and the 95% confidence interval. FIG. 17 right is a diagram illustrating discrimination of NPSLE based on C3 in serum + the area (area1) of the white connected components using the decision tree method.

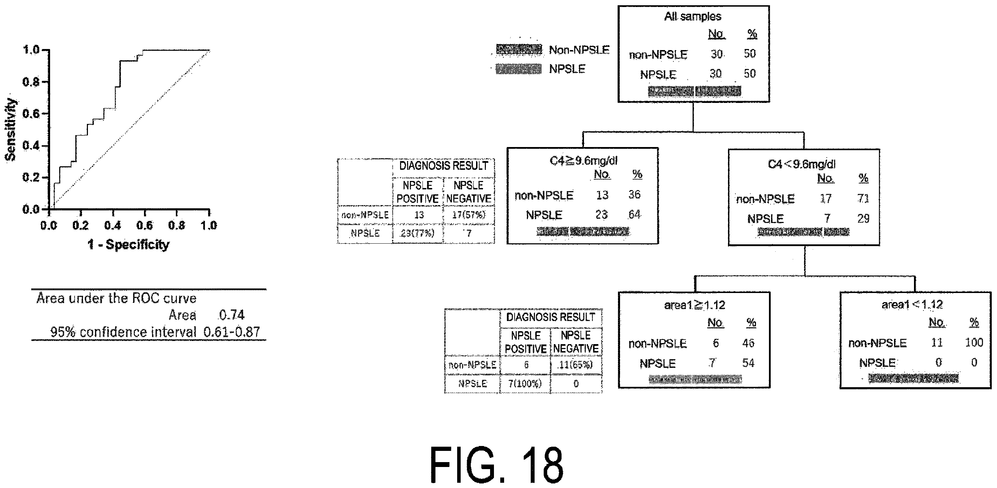

FIG. 18 left is a diagram illustrating an ROC curve of C4 in serum + the area (area1) of the white connected components, and the AUC and the 95% confidence interval. FIG. 18 right is a diagram illustrating discrimination of NPSLE based on C4 in serum + the area (area1) of the white connected components using the decision tree method.

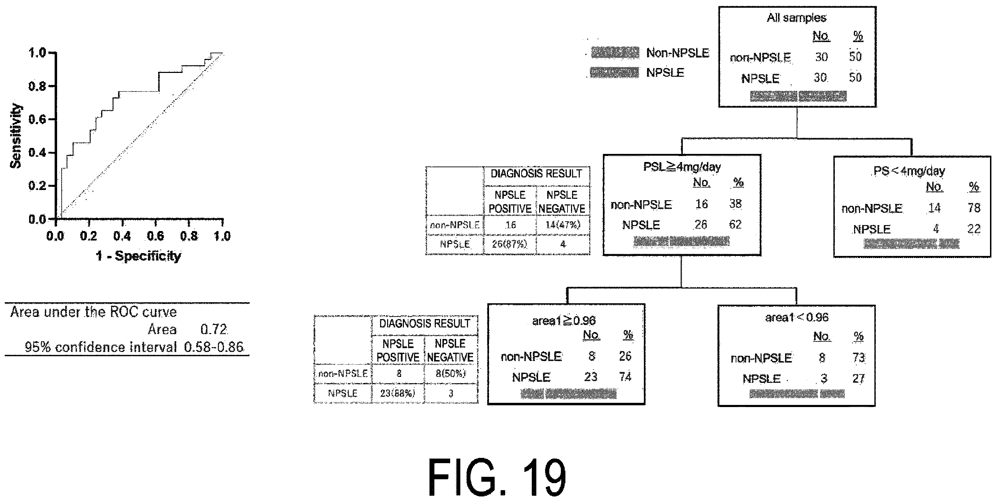

FIG. 19 left is a diagram illustrating an ROC curve of the amount of prednisolone (PSL) orally administered + the area (area1) of the white connected components, and the AUC and the 95% confidence interval. FIG. 19 right is a diagram illustrating discrimination of NPSLE based on the amount of prednisolone (PSL) orally administered + the area (area1) of the white connected components using the decision tree method.

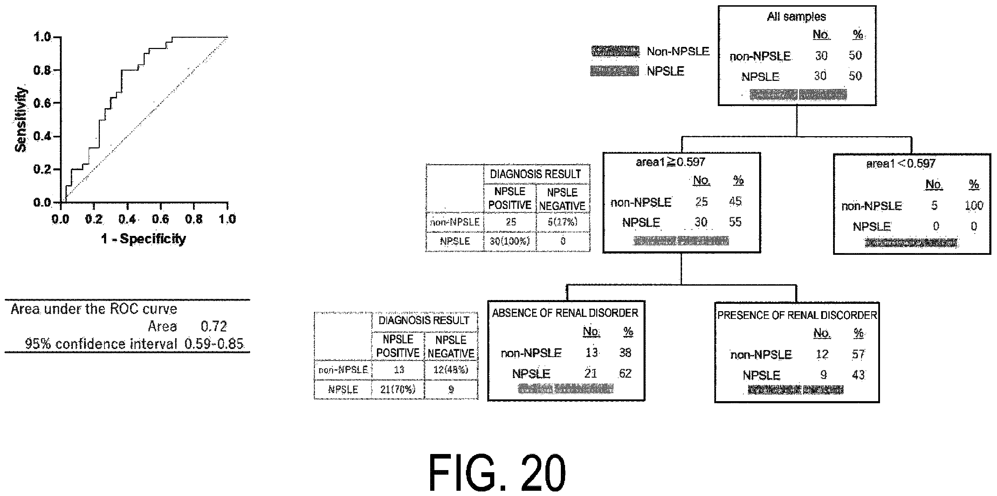

FIG. 20 left is a diagram illustrating an ROC curve of the presence or absence of renal disorder + the area (area1) of the white connected components, and the AUC and the 95% confidence interval. FIG. 20 right is a diagram illustrating discrimination of NPSLE based on the presence or absence of renal disorder + the area (area1) of the white connected components using the decision tree method.

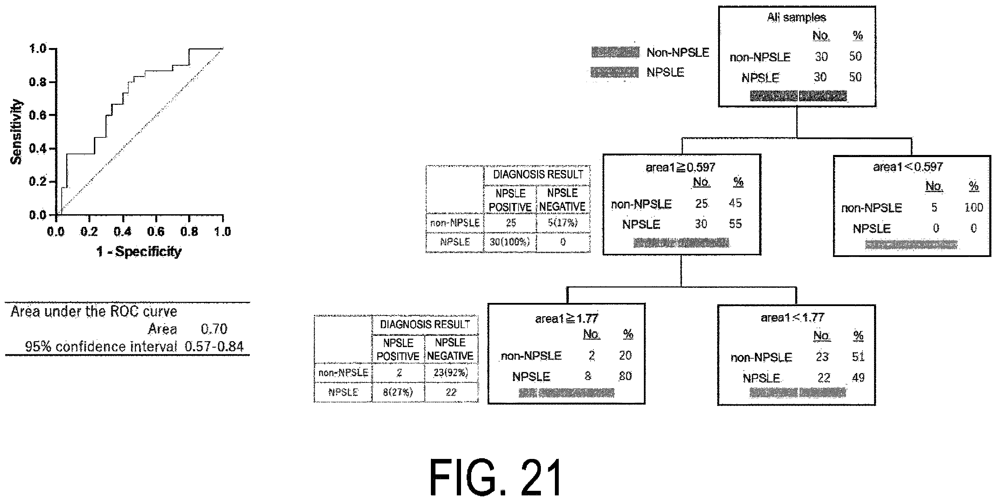

FIG. 21 left is a diagram illustrating an ROC curve of the number of platelets + the area (area1) of the white connected components, and the AUC and the 95% confidence interval. FIG. 21 right is a diagram illustrating discrimination of NPSLE based on the number of platelets + the area (area1) of the white connected components using the decision tree method.

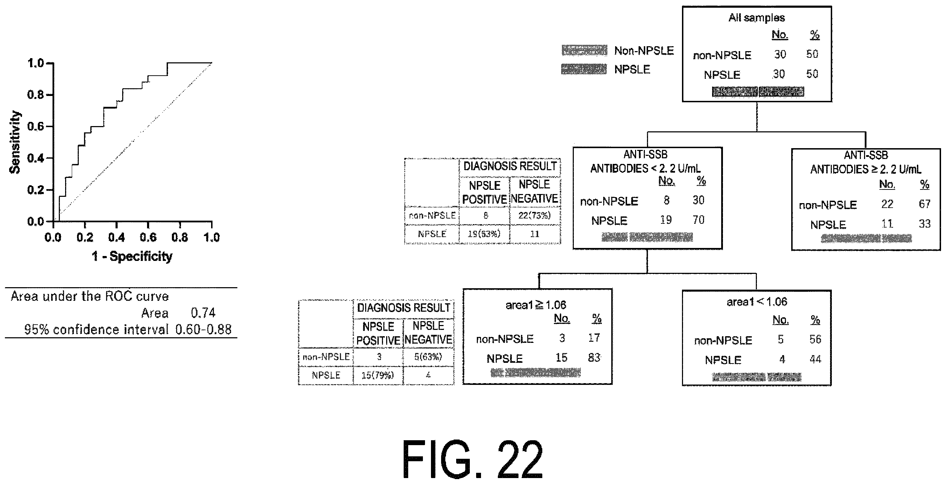

FIG. 22 left is a diagram illustrating an ROC curve of the amount of serum anti-SSB antibodies + the area (area1) of the white connected components, and the AUC and the 95% confidence interval. FIG. 22 right is a diagram illustrating discrimination of NPSLE based on the amount of serum anti-SSB antibodies + the area (area1) of the white connected components using the decision tree method.

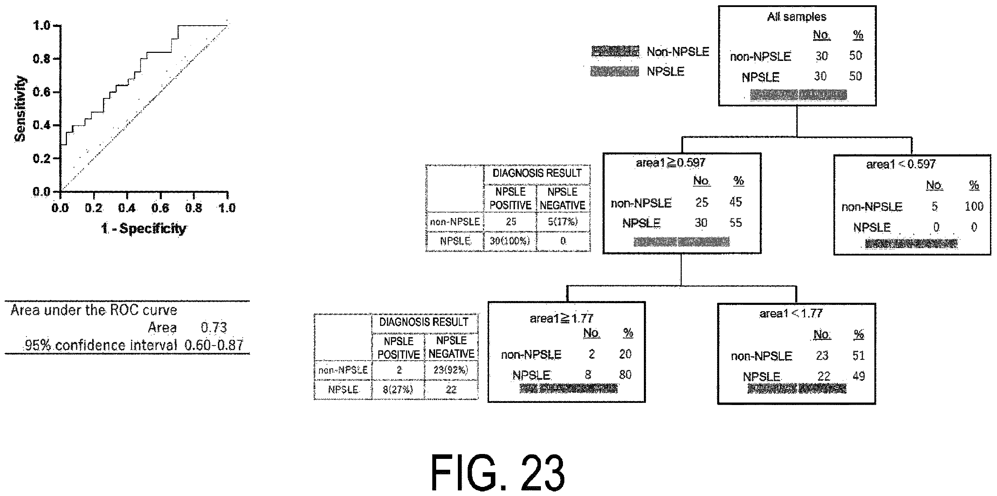

FIG. 23 left is a diagram illustrating an ROC curve of lupus anticoagulant + the area (area1) of the white connected components, and the AUC and the 95% confidence interval. FIG. 23 right is a diagram illustrating discrimination of NPSLE based on lupus anticoagulant + the area (area1) of the white connected components using the decision tree method.

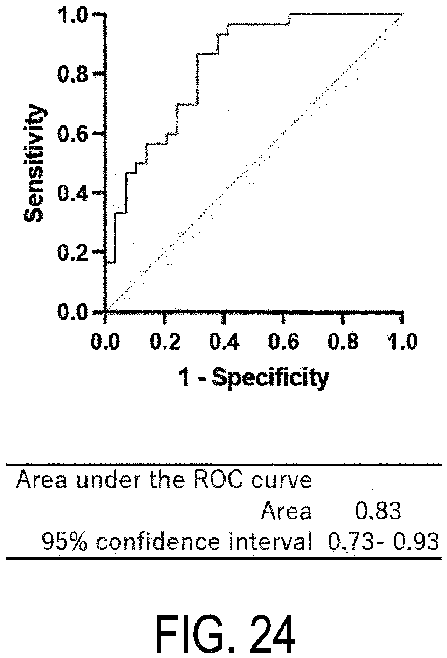

FIG. 24 is a diagram illustrating an ROC curves of the age + the history of cerebrovascular disorder + the serum complement value (CH50) + the perimeter (perimeter1) of the white connected components, and the AUC and the 95% confidence interval.

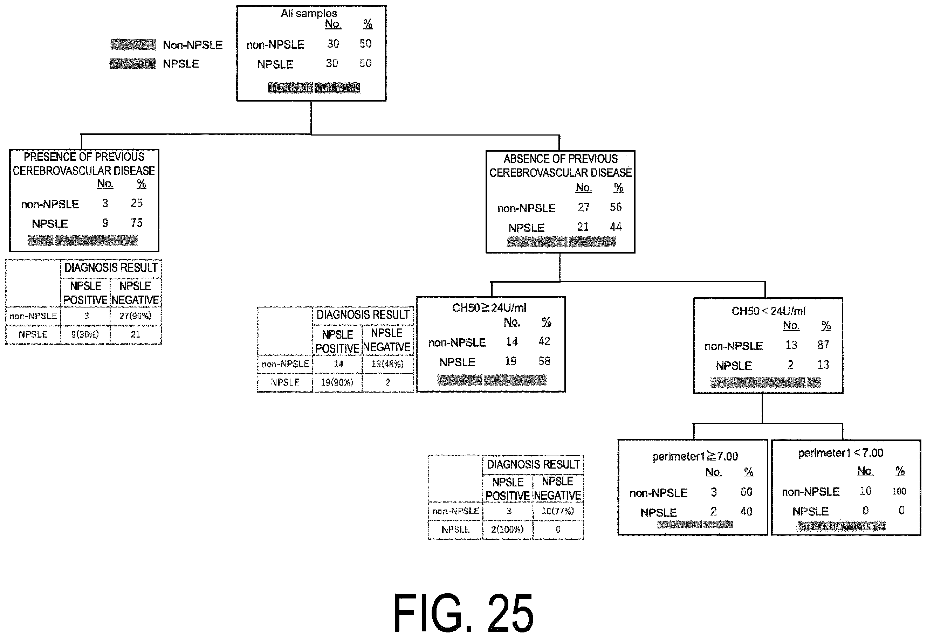

FIG. 25 is a diagram illustrating discrimination of NPSLE based on the age + the history of cerebrovascular disorder + the serum complement value (CH50) + the perimeter (perimeter1) of the white connected components using the decision tree method.

### Description of Embodiments

### 1. Method for obtaining an indicator for diagnosing whether a subject is an SLE patient or an NPSLE patient

The present invention provides a method for obtaining an indicator for diagnosing whether a subject is an SLE patient or an NPSLE patient. Specifically, the method according to an embodiment of the present invention includes:
(1) preparing head MRI images of SLE patients and NPSLE patients;
(2) gradually changing a color tone of each of the head MRI images;
(3) detecting and counting white connected components and black connected components generated in response to color tone changes;
(4) obtaining a scatter diagram of times of generation and disappearance of the white connected components and the black connected components based on the result in the detecting and counting (3);
(5) verifying whether there is a significant difference in any of image features between the SLE patients and the NPSLE patients, the image features being obtained from the scatter diagram; and
(6) identifying, as the indicator, an image feature in which a significant difference is recognized.

In step (1) of the present invention, images of the heads of SLE patients and NPSLE patients captured by magnetic resonance imaging (MRI) may be taken when the method of the present invention is performed, or may be taken during normal medical care or the like before the method of the present invention is performed. In addition, a third party other than the person performing the method of the present invention (for example, a medical worker of another hospital or the like) may capture and provide images. Furthermore, the images may be captured by MRI devices having different resolutions.

Imaging may be performed once or a plurality of times. In addition, the imaging method is not particularly limited and T1 weighted images, T2 weighted images, or the like may be used for head MRI images as long as the head MRI images allow a desired persistence diagram to be created in topological data analysis (which may hereinafter be abbreviated as "TDA") subsequent to step (2). Preferable head MRI images used in the method of the present invention include, for example, images captured by the fluid-attenuated inversion recovery (FLAIR) method which suppresses signals of cerebrospinal fluid and which is excellent in detection of lesions under the cerebral cortex which are difficult to distinguish from gray matter and sulci.

Step (1) of the present invention may include pre-processing head MRI images of SLE patients and NPSLE patients. The pre-processing is not particularly limited as long as the pre-processing facilitates persistent homology when the head MRI image is subjected to the persistent homology subsequent to the step (2). Specific examples of the pre-processing include color correction, contour enhancement, contrast correction, and trimming. A more specific example is skull stripping (removal of a skull from a brain image). This technique is useful for eliminating the effects of the contrast of the luminance of the skull in the brain image, which greatly varies among pieces of equipment. In performing skull stripping, a method known per se may be used, for example, a deep learning-based skull stripping program created in Buda et al., Comput. Biol. Medicine 109: 218-225 (2019). Further, in order to perform operations in the next step (2) and subsequent steps, for example, processing may be performed in which the luminance value of the brain image after skull stripping is normalized (the average is subtracted from the luminance value, and the result is divided by the standard deviation), the minimum value is subtracted from the normalized value to set the minimum value to 0, and then the luminance value of the region other than the brain is set to 0.

The "subject" to be tested in the present invention is not particularly limited, but is preferably an SLE patient or a person suspected of having SLE. In addition, the "subject" to be tested in the present invention is not limited to humans but includes mammals in general. Examples of such mammals include rodents (e.g., mice, rats, guinea pigs, gerbils, hamsters, etc.), primates (e.g., rhesus monkeys, cynomolgus monkeys, Japanese macaque, chimpanzees, etc.), experimental animals such as ferrets, rabbits, dogs, minipigs, etc., companion animals such as dogs, cats, etc., and farm animals such as cows, horses, pigs, sheep, etc.

In steps (2) to (4) of the present invention, topological data analysis (TDA) is used. In the present specification, topological data analysis (phase data analysis) refers to a technique of analyzing a feature of data by paying attention not to a statistical feature but to topological information of data (a form of data) with respect to large-scale multidimensional data. There are two types of techniques for TDA: persistent homology and mapper. Persistent homology is a technique that can capture geometric invariance and can quantitatively evaluate the details of data shape. Additionally, the mapper is a technique of representing high-dimensional data with a simple graph allowing geometric information to be recognized. In steps (2) to (4) of the present invention, persistent homology is used.

With respect to persistent homology, first, "homology" is a technique of representing a feature of an object by the number of m-dimensional holes (or islands) (m ≧ 0). As used herein, a "hole" (or "island") is an element of a homology group, a zero-dimensional hole (or island) is a connected component, a one-dimensional hole is a ring, and a two-dimensional hole is a cavity. Here, the number of holes (or islands) in each dimension (the number of connected components) is called the Betti number. Additionally, "persistent homology" is a technique for characterizing the transition of an m-dimensional hole in a target (here, a point cloud), and the persistent homology allows a feature related to a point layout to be examined. This technique includes gradually bulging each point in the target into a spherical (circular) shape, and during the process, determining the time when each hole (or each island) is generated (represented by the radius of the sphere (circle) at the time of generation) and the time when the hole (or the island) disappears (represented by the radius of the sphere (circle) at the time of disappearance).

As described above, persistent homology is generally a feature obtained by tracing the generation/disappearance of a hole (or island) appearing in the course of time-series changes with respect to columns of the graph (simplicial complexes). The time series of simplicial complexes is referred to as filtration. For example, the radius of each of the points of a given point cloud is gradually increased, and when circles overlap, the points are connected by a side. When a plurality of circles overlaps and a surface is constructed, then, in a graph including only points in the initial state, sides appear as time passes, and surfaces appear as time further passes. The change in the graph thus obtained is an example of filtration.

FIG. 1 is a diagram illustrating an image in which filtration is constructed. In persistent homology, each hole appearing in the filtration is associated with a pair of a generation time and a disappearance time. In FIG. 1, a hole is generated at time R = r2 and the hole disappears at time R = r3, and thus the persistent homology of the filtration represented by FIG. 1 is (r2, r3). In general, persistent homology is represented by a plurality of sets of (generation time, disappearance time) in order to handle changes in a complicated graph structure. In general, persistent homology is often represented by a persistence diagram (also referred to as a scatter diagram) in which the generation time is plotted on the horizontal axis and the disappearance time is plotted on the vertical axis.

In a case where a head MRI image is analyzed using persistent homology as in the present invention, a threshold for a color tone of the MRI image is gradually changed, and measurement is performed of thresholds (generation time and disappearance time) at which a white connected component (hereinafter referred to as a "hole" in some cases) is generated and disappears and thresholds (generation time and disappearance time) at which a black connected component (hereinafter referred to as an "island" in some cases) is generated and disappears (FIG. 2). The threshold may be a cutoff value of the luminance value. Next, a persistence diagram (scatter diagram) can be created by plotting the measured values with the generation time on the horizontal axis and the disappearance time on the vertical axis. The degree of change in the threshold value for the color tone is not particularly limited as long as a desired persistence diagram can be created, and may be in the range of 0 to 5, for example. For steps (2) to (4) described above, for example, a method described in a method known per se (Garside et al., PLOS ONE https://doi.org/10.1371/journal.pone.0217413 May 24, 2019) may be referred to.

In a case where persistent homology is used as the feature of the image, smoothing processing may be further applied to the persistence diagram. Various methods are conceivable for smoothing, and examples thereof include histogram formation, smoothing by kernel density estimation, and smoothing by a persistent intensity function.

In steps (5) and (6) of the present invention, an image feature (imaging biomarker) is acquired based on the persistence diagram (scatter diagram) created before step (5). For the image feature acquired, any significant difference between SLE patients and NPSLE patients is verified and identified. The image feature is not particularly limited as long as the image feature is acquired based on the persistence diagram (scatter diagram). For example, based on a known method (Henderson et al., Journal of the American Statistical Association 2020, 115: 625-635), an aggregate statistic for convex peel may be used as the image feature. To be more specific, for each of the white connected components and the black connected components, image features may be extracted and verified, such as the number and lifetime of points, and the center of gravity, perimeter, area, and filamentarity (a measure of the slenderness of the convex peel) of convex peel.

The verification and identification of any significant difference between the SLE patients and the NPSLE patients in steps (5) and (6) of the present invention may include, for example, performing logistic regression analysis for NPSLE based on the extracted image features for the white connected components and the black connected components and examining which of the image features has a significant relationship with NPSLE.

In the present invention, a specific procedure for generating the image features from the head MRI image data will be illustrated.
(1) One central image is extracted from a set of three-dimensionally-sliced head MRI images of each case of SLE and NPSLE.
(2) Each of the images is a grayscale image, which is converted to a size of 256 × 256.
(3) Skull stripping (removal of the skull from the brain image) is performed.
(4) The luminance value of the brain image after skull stripping is normalized (the average is subtracted from the luminance value, and the result is divided by the standard deviation), and the minimum value is subtracted from the normalized value to set the minimum value to 0, and then the luminance value of the region other than the brain is set to 0.
(5) With respect to the brain image of each case obtained before this step, a persistence diagram is created by using Dionysus 2, which is a software package of Python for calculating Persistent Homology, and by using the lower star filtration method, which is a technique suitable for images and lattice data.

### 2. Method for assisting in diagnosing whether a subject is an SLE patient or an NPSLE patient

One embodiment of the present invention provides a method for assisting diagnosis of whether a subject is an SLE patient or an NPSLE patient. Specifically, the method according to this embodiment of the present invention includes:
(1) preparing a head MRI image of a subject;
(2) gradually changing a color tone of the head MRI image;
(3) detecting and counting white connected components or black connected components generated in response to color tone changes;
(4) obtaining a scatter diagram of times of generation and disappearance of the white connected components and the black connected components based on results in the detecting (3);
(5) calculating a specific image feature from the scatter diagram; and
(6) determining whether the subject is an SLE patient or a NPSLE patient based on the calculated image feature.

For steps (1) to (4) included in the method for assisting diagnosis of whether a subject is an SLE patient or an NPSLE patient according to the present invention, all of the contents of " 1. Method for obtaining an indicator for diagnosing whether a subject is an SLE patient or an NPSLE patient" described above are incorporated herein by reference.

Determining whether the patient has NPSLE includes not only determining whether the subject has NPSLE or not (e.g., the patient has SLE but not NPSLE) but also includes assessing or calculating a likelihood or probability that the subject is NPSLE or a likelihood or probability that the subject is SLE (e.g., a patient having SLE but not NPSLE), which is useful in diagnosing whether the subject has NPSLE or SLE (e.g., the patient having SLE but not NPSLE). In addition, in the present specification, "assisting diagnosis" refers to providing information serving as an indicator for determining whether the subject has NPSLE, and means not including a step itself of making a diagnosis to determine whether the subject has NPSLE, which is a medical practice. Furthermore, in the present specification, "NPSLE" may include not only NPSLE as a disease but also an NPSLE patient. Similarly, "SLE" may include not only SLE as a disease but also an SLE patient.

The specific image feature calculated in step (5) of the present invention is not particularly limited as long as the image feature (imaging biomarker) can be acquired based on the persistence diagram (scatter diagram) created in step (4) and involves a significant difference between the image feature derived from SLE patients and the image feature derived from NPSLE patients. For example, among aggregate statistics for a convex peel of a persistence diagram (scatter diagram), an image feature may be used that involves a significant difference between the image feature derived from SLE patients and the image feature derived from NPSLE patients. To be more specific, for each of the white connected components and the black connected components, the following may be used as image features: the number and lifetime of points, and the center of gravity, perimeter (perimeter0: black), perimeter (perimeter1: white), area, and filamentarity (a measure of the slenderness of the convex peel) of convex peel. Examples of the preferable image feature in the present invention include, for example, the perimeter of a 90% convex peel for white connected components, and more preferably, the perimeter of a 95% convex peel for white connected components. In the present specification, the perimeter of the 90% convex peel (or the perimeter of the 95% convex peel) means the perimeter (arc length) of a convex peel containing 90% (or 95%) of plots in the created persistence diagram (scatter diagram) (FIG. 3). In the present invention, examples of the preferable image feature besides the perimeter of the convex peel include the area of the 90% convex peel for the white connected components and more preferably the area of the 95% convex peel for the white connected components. In the present specification, the area of the 90% convex peel (or the area of the 95% convex peel) means the area of a convex peel containing 90% (or 95%) of plots in the created persistence diagram (scatter diagram).

The calculated image feature in step (6) of the present invention may be compared with a reference value to determine whether the patient is an SLE patient or an NPSLE patient based on the image feature. The "reference value" used in the present invention may be the amount of the imaging biomarker of the present invention in a head MRI image of a patient having SLE but not NPSLE (which may hereinafter be referred to as a "control sample" in some cases). Alternatively, a value may be used that is set in advance from a measured value or a quantitative value of an imaging biomarker in a control sample. In this case, for example, a plurality of individuals is designated as a control group, and a median value, an average value, a mode value, or the like of measured values of the plurality of individuals can be adopted as a reference value.

The reference value may be a cutoff value. The "cutoff value" is a value that can satisfy both high diagnostic sensitivity (true positive ratio) and high diagnostic specificity (true negative ratio) when a disease is determined based on the value. For example, as a cutoff value, a value can be set that indicates a high positive rate in a population having NPSLE and a high negative rate in a population having SLE but not having NPSLE.

A method for calculating a cutoff value is well known in the art. The method includes, for example, measuring or quantifying the amount of the imaging biomarker of the present invention in each control sample (head MRI image), determining the diagnostic sensitivity and diagnostic specificity of the measured or quantified value, and based on these values, creating an ROC (Receiver Operating Characteristic) curve using analysis software (e.g., statistical analysis software JMP, statistical analysis software R, a scikit-learn package of Python, etc.). Then, a value is determined at which the diagnostic sensitivity and the diagnostic specificity are as close to 100% as possible, and this value can be used as a cutoff value. When two or more imaging biomarkers are used, or when one or more imaging biomarkers and one or more other biomarkers are used, the amounts of the respective biomarkers can be combined to generate an ROC curve.

Examples of other biomarkers include subject (patient) characteristics such as blood/cerebrospinal fluid tests and oral medicine. More specific examples include the age of the subject (patient) (when the subject is diagnosed as having NPSLE or MRI images of the subject are taken), the amount of prednisolone (PSL) orally administered to the subject (patient) (mg/day) (when the subject is diagnosed as having NPSLE or MRI images of the subject are taken), the amount of C3 in the serum of the subject (patient) (mg/dL) (when the subject is diagnosed as having NPSLE or MRI images of the subject are taken), the blood glucose level of the subject (patient) (mg/dL) (when the subject is diagnosed as having NPSLE or MRI images of the subject are taken), the amount of IL-6 in the cerebrospinal fluid of the subject (pg/mL) (when the subject is diagnosed as having NPSLE or MRI images of the subject are taken), the presence or absence of any previous cerebrovascular disease (when the subject is diagnosed as having NPSLE or MRI images of the subject are taken), serum complement value (CH50) (U/mL) in the subject (patient) (when the subject is diagnosed as having NPSLE or MRI images of the subject are taken), the amount of C4 in the subject (patient) (when the subject is diagnosed as having NPSLE or MRI images of the subject are taken), the presence or absence of renal disorder in the subject (patient) (when the subject is diagnosed as having NPSLE or MRI images of the subject are taken), the number of platelets in the subject (patient) (per µL), the amount of serum anti-SSB antibodies in the subject (patient) (U/mL), and serum lupus anticoagulant (reference value) in the subject (patient) (when the subject is diagnosed as having NPSLE or MRI images of the subject are taken).

In one embodiment, specific examples of the reference values that can be used in the present invention are as follows. In the diagnosis of SLE and NPSLE, the sensitivity, specificity, and the AUC (Area Under the Curve) are respectively 90%, 37%, and 0.65 for the perimeter (perimeter1) of the 95% convex peel for the white connected components, which are image features that can be acquired from a persistence diagram (cutoff value of perimeter1 ≥ 5.34).

In the diagnosis of SLE and NPSLE, the AUC is 0.75 when the perimeter (perimeter1) of the 95% convex peel for the white connected components is combined with the age of the subject (patient) (when NPSLE is diagnosed or MRI imaging). When SLE and NPSLE are branched off by the decision tree method, at the cutoff value of perimeter1 ≥ 5.34, the sensitivity is 90%, and the specificity is 37%. When the group of patients distinguished as having NPSLE in the first branching is branched again by the decision tree method, at the cutoff value of less than 44 years of age, the sensitivity is 70%, and the specificity is 63%.

In the diagnosis of SLE and NPSLE, the AUC is 0.71 when the perimeter (perimeter1) of the 95% convex peel for the white connected components is combined with the amount of prednisolone orally administered to the subject (patient). When SLE and NPSLE are branched off by the decision tree method, at the cutoff value of the amount of prednisolone ≥ 4 mg/day, the sensitivity is 87%, and the specificity is 43%. When the group of patients distinguished as having NPSLE in the first branching is branched again by the decision tree method, at the cutoff value of perimeter1 ≥ 5.34, the sensitivity is 92%, and the specificity is 41%.

In the diagnosis of SLE and NPSLE, the AUC is 0.73 when the perimeter (perimeter1) of the 95% convex peel for the white connected components is combined with C3 in the serum of the subject (patient) (mg/dL). When SLE and NPSLE are branched off by the decision tree method, at the cutoff value of perimeter1 ≥ 5.34, the sensitivity is 90%, and the specificity is 37%. When the group of patients distinguished as having NPSLE in the first branching is branched again by the decision tree method, at the cutoff value of C3 in serum ≥ 91.4 mg/dL, the sensitivity is 26%, and the specificity is 100%.

In the diagnosis of SLE and NPSLE, the AUC is 0.73 when the perimeter (perimeter1) of the 95% convex peel for the white connected components is combined with the blood glucose level of the subject (patient) (mg/dL). When SLE and NPSLE are branched off by the decision tree method, at the cutoff value of perimeter1 ≥ 5.34, the sensitivity is 90%, and the specificity is 37%. When the group of patients distinguished as having SLE in the first branching is branched again by the decision tree method, at the cutoff value of blood glucose level ≥ 92 mg/dL, the sensitivity is 100%, and the specificity is 73%.

In the diagnosis of SLE and NPSLE, the AUC is 0.74 when the perimeter (perimeter1) of the 95% convex peel for the white connected components is combined with IL-6 in the cerebrospinal fluid of the subject (patient) (pg/mL). When SLE and NPSLE are branched off by the decision tree method, at the cutoff value of IL-6 in the cerebrospinal fluid ≥ 22.8 pg/mL, the sensitivity is 23%, and the specificity is 80%. When the group of patients distinguished as having SLE in the first branching is branched again by the decision tree method, at the cutoff value of perimeter1 ≥ 5.60, the sensitivity is 87% and the specificity is 42%.

A suitable cutoff value for the perimeter (perimeter1) of the 95% convex peel for the white connected components, which is the imaging biomarker used in the present invention, may be perimeter1 ≥ 5.34. Examples of the values are as follows: for a combination with the age of the subject (patient), for example, the cutoff value of less than 44 years of age and perimeter1 ≥ 5.34, for a combination with the amount of prednisolone orally administered to the subject (patient), the cutoff value of the amount of prednisolone ≥ 4 mg/day and perimeter1 ≥ 5.34, for a combination with C3 in the subject (patient) (mg/dL), the cutoff value of C3 in serum ≥ 91.4 mg/dL and perimeter1 ≥ 5.34, for a combination with the blood glucose level of the subject (patient) (mg/dL), the cutoff value of blood glucose level ≥ 92 mg/dL and perimeter1 ≥ 5.34, and for a combination with IL-6 in the cerebrospinal fluid of the subject (patient) (pg/mL), the cutoff value of IL-6 in cerebrospinal fluid ≥ 22.8 pg/mL and perimeter1 ≥ 5.60. However, these numerical values may vary depending on the sample size and other conditions, and the present invention is not limited to these numerical values.

In one embodiment, further specific examples of the reference values that can be used in the present invention are as follows. In the diagnosis of SLE and NPSLE, the sensitivity, specificity, and AUC (Area Under the Curve) are respectively 100%, 17%, and 0.66 for the area (area1) of the 95% convex peel for the white connected components, which is an image feature that can be acquired from a persistence diagram (cutoff value of area1 ≥ 0.597).

The AUC is 0.82 for the combination of the area (area1) of the 95% convex peel for the white connected components, the history of a cerebrovascular disease of the subject (patient) (when the subject is diagnosed as having NPSLE or MRI images of the subject are taken), serum complement value (CH50) in the subject (patient) (U/mL) (when the subject is diagnosed as having NPSLE or MRI images of the subject are taken), and the age of the subject (patient) (when the subject is diagnosed as having NPSLE or MRI images of the subject are taken). When SLE and NPSLE are branched off by the decision tree method, at the cutoff value of the previous presence of a cerebrovascular disease, the sensitivity is 30%, and the specificity is 90%. When the group of patients distinguished as having NPSLE in the first branching is branched again by the decision tree method, at the cutoff value of less than 48 years of age, the sensitivity is 56%, and the specificity is 100%. Additionally, when the group of patients distinguished as having SLE in the first branching is branched again by the decision tree method, at the cutoff value of CH50 ≥ 24 U/mL, the sensitivity is 90%, and the specificity is 48%. When the group of patients distinguished as having NPSLE in the second branching is branched again by the decision tree method, at the cutoff value of less than 53 years of age, the sensitivity is 89%, and the specificity is 43%. When the group of patients distinguished as having NPSLE in the third branching is branched again by the decision tree method, at the cutoff value of area1 ≥ 0.802, the sensitivity is 88%, and the specificity is 50%.

In the diagnosis of SLE and NPSLE, the AUC is 0.72 when the area (area1) of the 95% convex peel for the white connected components is combined with the serum complement value (CH50) of the subject (patient) (U/mL) (when the subject is diagnosed as having NPSLE or MRI images of the subject are taken). When SLE and NPSLE are branched off by the decision tree method, at the cutoff value of area1 ≥ 0.597, the sensitivity is 100%, and the specificity is 17%. When the group of patients distinguished as having NPSLE in the first branching is branched again by the decision tree method, at the cutoff value of CH50 ≥ 20.7 U/mL, the sensitivity is 93%, and the specificity is 32%.

In the diagnosis of SLE and NPSLE, the AUC is 0.73 when the area (area1) of the 95% convex peel for the white connected components is combined with the age of the subject (patient) (when the subject is diagnosed as having NPSLE or MRI images of the subject are taken). When SLE and NPSLE are branched off by the decision tree method, at the cutoff value of area1 ≥ 0.597, the sensitivity is 100%, and the specificity is 17%. When the group of patients distinguished as having NPSLE in the first branching is branched again by the decision tree method, at the cutoff value of less than 44 years of age, the sensitivity is 70%, and the specificity is 60%.

In the diagnosis of SLE and NPSLE, the AUC is 0.73 when the area (area1) of the 95% convex peel for the white connected components is combined with C3 in the serum of the subject (patient)(mg/dL). When SLE and NPSLE are branched off by the decision tree method, at the cutoff value of area1 ≥ 0.597, the sensitivity is 100%, and the specificity is 17%. When the group of patients distinguished as having NPSLE in the first branching is branched again by the decision tree method, at the cutoff value of C3 in serum ≥ 67.7 mg/dL, the sensitivity is 60%, and the specificity is 72%.

In the diagnosis of SLE and NPSLE, the AUC is 0.74 when the area (area1) of the 95% convex peel for the white connected components is combined with C4 in the serum of the subject (patient) (mg/dL). When SLE and NPSLE are branched off by the decision tree method, at the cutoff value of C4 in serum ≥ 9.6 mg/dL, the sensitivity is 77%, and the specificity is 57%. When the group of patients distinguished as having SLE in the first branching is branched again by the decision tree method, at the cutoff value of area1 ≥ 1.12, the sensitivity is 100%, and the specificity is 65%.

In the diagnosis of SLE and NPSLE, the AUC is 0.72 when the area (area1) of the 95% convex peel for the white connected components is combined with the amount of prednisolone (PSL) orally administered to the subject (patient). When SLE and NPSLE are branched off by the decision tree method, at the cutoff value of the amount of prednisolone ≥ 4 mg/day, the sensitivity is 87%, and the specificity is 47%. When the group of patients distinguished as having NPSLE in the first branching is branched again by the decision tree method, at the cutoff value of area1 ≥ 0.96, the sensitivity is 88%, and the specificity is 50%.

In the diagnosis of SLE and NPSLE, the AUC is 0.72 when the area (area1) of the 95% convex peel for the white connected components is combined with the presence or absence of renal disorder in the subject (patient). When SLE and NPSLE are branched off by the decision tree method, at the cutoff value of area1 ≥ 0.597, the sensitivity is 100%, and the specificity is 17%. When the group of patients distinguished as having NPSLE in the first branching is branched again by the decision tree method, at the cutoff value of absence of renal disorder, the sensitivity is 70%, and the specificity is 48%.

In the diagnosis of SLE and NPSLE, the AUC is 0.70 when the area (area1) of the 95% convex peel for the white connected components is combined with the number of platelets in the subject (patient). When SLE and NPSLE are branched off by the decision tree method, at the cutoff value of area1 ≥ 0.597, the sensitivity is 100%, and the specificity is 17%. When the group of patients distinguished as having NPSLE in the first branching is branched again by the decision tree method, at the cutoff value of area1 ≥ 1.77, the sensitivity is 27%, and the specificity is 92%.

In the diagnosis of SLE and NPSLE, the AUC is 0.74 when the area (area1) of the 95% convex peel for the white connected components is combined with the amount of serum anti-SSB antibodies in the subject (patient) (U/mL). When SLE and NPSLE are branched off by the decision tree method, at the cutoff value of the amount of serum anti-SSB antibodies < 2.2 U/mL, the sensitivity is 63%, and specificity is 73%. When the group of patients distinguished as having NPSLE in the first branching is branched again by the decision tree method, at the cutoff value of area1 ≥ 1.06, the sensitivity is 79%, and the specificity is 63%.

In the diagnosis of SLE and NPSLE, the AUC is 0.73 when the area (area1) of the 95% convex peel for the white connected components is combined with the serum lupus anticoagulant in the subject (patient). When SLE and NPSLE are branched off by the decision tree method, at the cutoff value of area1 ≥ 0.597, the sensitivity is 100%, and the specificity is 17%. When the group of patients distinguished as having NPSLE in the first branching is branched again by the decision tree method, at the cutoff value of area1 ≥ 1.77, the sensitivity is 27%, and the specificity is 92%.

The AUC is 0.83 when the perimeter (perimeter1) of the 95% convex peel for the white connected components is combined with the history of a cerebrovascular disease of the subject (patient) (when the subject is diagnosed as having NPSLE or MRI images of the subject are taken), the serum complement value (CH50) of the subject (patient) (U/mL) (when the subject is diagnosed as having NPSLE or MRI images of the subject are taken), and the age of the subject (patient) (when the subject is diagnosed as having NPSLE or MRI images of the subject are taken). When SLE and NPSLE are branched off by the decision tree method, at the cutoff value of the previous presence of a cerebrovascular disease, the sensitivity is 30%, and the specificity is 90%. Additionally, when the group of patients distinguished as having SLE in the first branching is branched again by the decision tree method, at the cutoff value of CH50 ≥ 24 U/mL, the sensitivity is 90%, and the specificity is 48%. When the group of patients distinguished as having SLE in the second branching is branched again by the decision tree method, at the cutoff value of perimeter1 ≥ 7.00, the sensitivity is 100%, and the specificity is 77%.

A suitable cutoff values for the area (area1) of the 95% convex peel for the white connected components, which is an imaging biomarker used in the present invention, may be area1 ≥ 0.597. Additionally, in the case where the history of a cerebrovascular disease of the subject (patient) (when the subject is diagnosed as having NPSLE or MRI images of the subject are taken) is combined with the serum complement value (CH50) of the subject (patient) (U/mL) (when the subject is diagnosed as having NPSLE or MRI images of the subject are taken), and the age of the subject (patient) (when the subject is diagnosed as having NPSLE or MRI images of the subject are taken), suitable cutoff values include absence of a previous cerebrovascular disease, CH50 ≥ 24 U/mL, less than 53 years of age, and area1 ≥ 0.802, for example. In a combination with the serum complement value (CH50) of the subject (patient) (U/mL), suitable cutoff values include, for example, area1 ≥ 0.597, and CH50 ≥ 20.7 U/mL. In a combination with the ages of subject (patient), suitable cutoff values include, for example, less than 44 years of age and area1 ≥ 0.597. In a combination with C3 in the serum of the subject (patient) (mg/dL), suitable cutoff values include, for example, C3 in serum ≥ 67.7 mg/dL, and area1 ≥ 0.597. In a combination with C4 in the serum of the subject (patient) (mg/dL), suitable cutoff values include, for example, C4 in serum ≥ 9.6 mg/dL, and area1 ≥ 1.12. In a combination with the amount of prednisolone (PSL) orally administered to the subject (patient), suitable cutoff values include, for example, the amount of prednisolone ≥ 4 mg/day, and area1 ≥ 0.96. In a combination with the presence or absence of renal disorder of the subject (patient), suitable cutoff values include, for example, absence of renal disorder, and area1 ≥ 0.597. In a combination with the number of platelets in the subject (patient), suitable cutoff values include, for example, area1 ≥ 1.77 and area1 ≥ 0.597. In a combination with the amount of serum anti-SSB antibodies in the subject (patient) (U/mL), suitable cutoff values include, for example, the amount of serum anti-SSB antibodies < 2.2 U/mL, and area1 ≥ 1.06. In a combination with the serum lupus anticoagulant in the subject (patient), suitable cutoff values include, for example, area1 ≥ 1.77, and area1 ≥ 0.597.

Another suitable cutoff value for the perimeter (perimeter1) of the 95% convex peel for the white connected components, which is an imaging biomarker used in the present invention, is perimeter1 ≥ 7.00. In the case where the history of a cerebrovascular disease of the subject (patient) (when the subject is diagnosed as having NPSLE or MRI images of the subject are taken) is combined with the serum complement value (CH50) of the subject (patient) (U/mL) (when the subject is diagnosed as having NPSLE or MRI images of the subject are taken), and the age of the subject (patient) (when the subject is diagnosed as having NPSLE or MRI images of the subject are taken), suitable cutoff values include the presence of a previous cerebrovascular disease, CH50 ≥ 24 U/mL, and perimeter1 ≥ 7.00, for example. These numerical values may vary depending on the sample size and other conditions, and the present invention is not limited to these numerical values.

Additionally, whether the subject is NPSLE (whether the subject has NPSLE or SLE (e.g., the subject has SLE but not NPSLE)) can be determined by a classifier. Algorithms used in such classifiers include, for example, the support vector machine algorithm, logistic regression algorithm, polynomial logistic regression algorithm, Fisher's linear discriminant algorithm, quadratic classifier algorithm, perceptron algorithm, k-nearest neighbor algorithm, random forest algorithm, decision tree algorithm, naive Bayes algorithm, a combination of these algorithms, and the like.

The reference value may be a branch point reference value set by a decision tree analysis using the algorithm described above. For example, in each control sample, a branch point can be determined by determining the amount of the imaging biomarker of the present invention and optionally the amount of other biomarkers, performing a decision tree analysis, and using impurity of data represented by a reference measure such as a Gini index, entropy, or a chi-square statistic to create variables (for example, the perimeter (perimeter1) of the 95% convex peel for the white connected components, the age of the subject (patient), the amount of prednisolone orally administered to the subject (patient), the amount of C3 in serum of the subject (patient), the blood glucose level of the subject (patient), the amount of IL-6 in the cerebrospinal fluid of the subject, the area (area1) of the 95% convex peel for the white connected components, the presence or absence of any previous cerebrovascular disease in the subject (patient), serum complement value (CH50) in the subject (patient), the amount of C4 in serum of the subject (patient), the presence or absence of renal disorder in the subject (patient), the number of platelets in the subject (patient), the amount of serum anti-SSB antibodies in the subject (patient), and serum lupus anticoagulant in the subject (patient) and the like) and reference values therefor. The decision tree analysis can be performed by a method known per se, for example, a method using software for decision tree analysis (statistical analysis software JMP, statistical analysis software R, rpart package, and the like).

As a result of comparison of the amount of the imaging biomarker of the present invention (or optionally a combination of the amount of the imaging biomarker of the present invention and the amount of other biomarkers), the subject (patient) can be assessed as NPSLE positive (having NPSLE) in a case where, for example, in a sample (e.g., a head MRI image or the like) derived from the subject (patient), the imaging biomarker of the present invention is measured as a statistically significantly higher value than in a control sample (e.g., a head MRI image or the like) or as a value equal to or higher than the reference value, and the subject (patient) can be assessed as NPSLE negative (likely to be NPSLE negative) (in other expressions, evaluated as non-NPSLE (likely to be non-NPSLE) or evaluated as SLE but as non-NPSLE (likely to be SLE but non-NPSLE) in a case where the imaging biomarker of the present invention does not exhibit a statistically significant difference from the control sample (e.g., a head MRI image or the like) or is measured as a statistically significantly lower value than in the control sample or as a value lower than the reference value.

### 3. A treatment or prevention method for an SLE patient or an NPSLE patient

As a result of the method of the present invention described in 2. above, in a case where, for example, the subject (patient) determined to have NPSLE or determined to have a possibility of having NPSLE, NPSLE can be treated by selecting or determining a therapeutic agent for NPSLE to be administered to the subject based on the result of the determination or the like, and administering a therapeutically effective amount of the therapeutic agent to the subject. As a result of the method of the present invention, in a case where, for example, the subject (patient) is determined to have SLE but not have NPSLE (SLE but non-NPSLE), SLE can be treated by selecting or determining a therapeutic agent for SLE to be administered to the subject based on the result of the determination or the like, and administering a therapeutically effective amount of the therapeutic agent to the subject. In the present specification, the "therapeutic agent" includes not only a medicament intended for radical treatment of NPSLE or SLE, but also, for example, a medicament intended to suppress the progression of these diseases or a medicament intended to alleviate the symptoms.

A specifically provided method for treating or preventing NPSLE (or SLE) of one embodiment of the present invention includes:
(1) preparing a head MRI image of a subject;
(2) gradually changing a color tone of the head MRI image;
(3) detecting and counting white connected components or black connected components generated in response to color tone changes;
(4) obtaining a scatter diagram of times of generation and disappearance of the white connected components and the black connected components based on a result of the detecting and counting (3);
(5) calculating a specific image feature from the scatter diagram;
(6) determining, from the calculated image feature, whether the subject is an SLE patient or an NPSLE patient; and
(7) administering a therapeutic agent for NPSLE (or SLE) to the subject determined to have NPSLE (or SLE) based on the determination result in the determining (6).

Examples of therapeutic agents for SLE and/or NPSLE include corticosteroids (e.g., prednisone, prednisolone, methylprednisolone, hydrocortisone, etc.), disease-modifying antirheumatic drugs (DMARDs) (e.g., methotrexate, salazosulfapyridine, bucillamine, leflunomide, tacrolimus, etc.), anti-malarial drugs (e.g., hydroxychloroquine, chloroquine, etc.), immunosuppressive drugs (e.g., cyclophosphamide, azathioprine, mycophenolate mofetil, methotrexate, etc.), non-steroidal anti-inflammatory drugs (NSAIDs) (e.g., acetylsalicylic acid, ibuprofen, naproxen, indomethacin, nabumetone, celecoxib, etc.), antihypertensive drugs (e.g., calcium channel blocker (e.g., amlodipine, nifedipine, etc.), diuretics (e.g., furosemide), statins (e.g., atorvastitin, fluvastatin, lovastatin, pitavastatin, pravastatin, rosuvastatin, simvastatin, etc.), anti-B-cell drugs (e.g., anti-CD20 antibodies (e.g., ritsuximab, etc.), anti-CD22 antibodies, etc.), anti-BLys antibodies (e.g., Belimumab, blisibimod, etc.), anti-T-lymphocyte antibodies (e.g., abatacept, etc.), type 1 interferon receptor antagonists (e.g., anifrolumab, etc.), anti-CD4 antibodies (e.g., ligerimodo, tregalizumab, etc.), anticoagulants (e.g., heparin, warfan, etc.), vitamin D supplements, and the like. However, the present invention is not limited to these drugs and any other common drugs may be used. The above-mentioned therapeutic agents may be used in appropriate combination according to the symptoms (SLE or NPSLE) of patients.

The therapeutic agent may be administered orally or parenterally as a pharmaceutical composition in an appropriate dosage form by independently taking the active ingredient or mixing the active ingredient with a pharmaceutically acceptable carrier, excipient, diluent, or the like. Examples of the composition for oral administration include solid or liquid dosage forms, specifically tablets (including sugar-coated tablets and film-coated tablets), pills, granules, powders, capsules (including soft capsules), syrups, emulsions, suspensions and the like. On the other hand, as the composition for parenteral administration, for example, injections, suppositories and the like are used, and the injections may include dosage forms such as intravenous injections, subcutaneous injections, intradermal injections, intramuscular injections, drip injections and the like. Additionally, the dose of the therapeutic agent can be appropriately set depending on various conditions such as the kind of the compound, the symptom, age, body weight, and drug acceptability of the target of administration.

For the treatment or prevention method of the present invention, the contents of " 1. Method for obtaining an indicator for diagnosing whether a subject is an SLE patient or an NPSLE patient" and "2. Method for assisting diagnosis of whether a subject is an SLE patient or NPSLE patient" are all incorporated herein by reference.

### 4. Computer program for predicting an SLE patient or an NPSLE patient

In one embodiment, a diagnosis assisting method of the present invention may be executed using a computer and a program executed by the computer. Thus, yet another embodiment provides a computer program for predicting whether a subject is an SLE patient or an NPSLE patient, the computer program causing a computer to function as:
a reception unit (P1) configured to receive head MRI image data of a prediction target;
a calculation unit (P2) configured to
   (i) gradually change a color tone of the received head MRI image data,
   (ii) detect and count white connected components or black connected components generated in response to color tone changes,
   (iii) obtain a scatter diagram of times of generation and disappearance of the white connected components and the black connected components based on a result of the detecting and counting,
   (iv) calculate a specific image feature from the scatter diagram, and
   (v) calculate at least one of a probability that the subject is an SLE patient or a probability that the subject is an NPSLE patient; and
an output unit (P3) configured to output a calculation result obtained by the calculating unit.

The reception unit P1 is a program portion for receiving, as an input data group, the head MRI image data of the prediction target from an external input device, a computer on which the program of the present invention is executed, or an external computer. The input data group may have a program configuration that directly receives the input data group transmitted by the operator. In addition, the input data group may have a program configuration of accessing a known storage device (SSD, HDD, DVD-ROM, CD-ROM, or the like) or an external database and capturing data based on input of a data name by an operator.

The external device for inputting the input data group to the reception unit P1 is typically an external computer accessibly connected via the Internet to a computer executing the program of the present invention, but may be a memory (USB memory) or any of various data reading devices connected via an interface such as a USB to the computer that executes the program of the present invention. The reception unit P1 is configured to receive an input data group from a computer or various pieces of external equipment and deliver the input data group to the calculation unit P2.

The calculation unit P2 is a program portion for executing the above-described diagnostic assistance method of the present invention in order to process the input data group transferred from the reception unit P1 and calculate at least one of the probability of having SLE or the probability of having NPSLE. The process of the calculation is as described in P2 (i) to (v). The probability of having SLE or the probability of having NPSLE obtained by the calculation is delivered to the output unit P3.

The output unit P3 is a program portion for outputting the probability of having SLE or the probability of having NPSLE obtained by the calculating unit P2. The manner of outputting the calculation result from the calculation unit P2 may be, for example, outputting the probability of having SLE or the probability of having NPSLE, or outputting whether the subject has SLE or NPSLE. The predetermined output destination to which the prediction result is output is not particularly limited, and may be a predetermined output destination such as any of various image display devices, any of various print output devices, or a computer capable of communicating with a computer that executes the program of the present invention.

The computer program of the present invention incorporates all the contents of "1. Method for obtaining an indicator for diagnosing whether a subject is an SLE patient or an NPSLE patient", "2. Method for assisting diagnosis of whether a subject is an SLE patient or an NPSLE patient", and "3. Treatment or prevention method for an SLE patient or an NPSLE patient" described above.

Hereinafter, the present invention will be more specifically described by taking examples, but the present invention is not limited to the examples below.

### Examples

### Example 1: Acquisition of the indicator of the present invention and construction of the method of the present invention (patient and image)

In the present example, among SLE patients who visited the Department of Immunology and Rheumatology of Nagasaki University Hospital between Jan. 1, 2005 and Aug. 31, 2020, patients diagnosed as having NPSLE were set as an NPSLE group, and SLE patients without Neuropsychiatric symptoms were set as a non-NPSLE group, and during normal medical care, head MRI FLAIR images were taken of 30 patients of the NPSLE group and of the non-NPSLE group and compared with one another.

The median age of the non-NPSLE group was 44.5 years of age (interquartile range: 30.8 to 54.3 years of age) and the median age of the NPSLE group was 39.5 years of age (interquartile range: 28.5 to 47.3 years of age). Also, the non-NPSLE group included 25 women (83%), and the NPSLE group included 22 women (73%). The head MRI images of the NPSLE group were obtained when the patients were diagnosed as having NPSLE, or when the patients visited the Department of Immunology and Rheumatology of Nagasaki University Hospital first. The head MRI images of the non-NPSLE group were obtained when the patients were diagnosed as SLE or when the patients visited the Department of Immunology and Rheumatology of Nagasaki University Hospital first. The head MRI images were not captured by a single piece of equipment, but were captured by a plurality of types of equipment. Images taken in other institutes were also used.

### [NPSLE Group]

### (Selection Criteria)

Patients satisfying any one of the following:
(i) NPSLE patients who underwent medical examination for definitive diagnosis in the Department of Immunology and Rheumatology of Nagasaki University Hospital between Jan. 1, 2005 and Aug. 31, 2020
(ii) NPSLE patients who received a definitive diagnosis by the other institutes, were referred to the Department of Immunology and Rheumatology of Nagasaki University Hospital, and underwent the first medical examination in Nagasaki University Hospital between Jan. 1, 2005 and Aug. 31, 2020.

### <Reasons for the Setting>

(i) and (ii) to use information and specimens obtained in the normal medical care.

### (Exclusion Criteria)

(i) patients who underwent no head MRI
(ii) patients determined by the persons responsible for the study to be unsuitable as subjects to be studied.

### <Reasons for the Setting>

(ii) to use head MRI images obtained in the normal medical care.

### [Non-NPSLE Group]

### (Selection Criteria)

(i) SLE patients who underwent medical examination for definitive diagnosis in the Department of Immunology and Rheumatology of Nagasaki University Hospital between Jan. 1, 2005 and Aug. 31, 2020.
(ii) SLE patients who received a definitive diagnosis by other institutes, were referred to the Department of Immunology and Rheumatology of Nagasaki University Hospital, and underwent the first medical examination in the hospital between Jan. 1, 2005 and Aug. 31, 2020.

### <Reasons for the Setting>

(i) and (ii) to use information and specimens obtained in the normal medical care.

### (Exclusion Criteria)

(i) patients exhibiting no neuropsychiatric symptoms.
(ii) patients who underwent no head MRI.
(iii) patients determined by the persons responsible for the study to be unsuitable as subjects to be studied.

### <Reasons for the Setting>

(i) To exclude NPSLE patients.
(ii) to use head MRI images obtained in the normal medical care.

### Example 2: Acquisition of the indicator of the present invention and construction of the method of the present invention (each analysis)

### [Statistical Analysis]

Statistical analyses were performed using JMP (trade name) Pro15 software (SAS Institute, Cary, NC) and Graphpad Prism9 (Graphpad software, San Diego, CA.). A case of statistical significance at a significance level of 5% was regarded as significant.

### [Topological Data Analysis]

The present example refers to the technique of constructing an image feature by topological data analysis, provided by Garside et al. (2019) as described above. Garside et al. (2019) discriminated a diabetic retinopathy patient from a healthy person by using, as a feature for machine learning, an image feature extracted from a retinal blood vessel image by TDA. The image feature of the TDA is acquired by describing changes in a two-dimensional lattice obtained by filtering various luminance values using the cutoff of the luminance values.

With reference to Garside et al. (2019) mentioned above, the present example used, as an image feature, an aggregate statistic (Henderson et al. (2020) mentioned above) for a convex peel of a persistence diagram recording luminance value cutoffs corresponding to appearance and disappearance of two topological features of black connected components and white connected components (holes) obtained through filtering. In the present example, for each of the black connected components and the white connected components, the number and lifetime of points, and the center of gravity, the perimeter, area, and filamentarity (a measure of slenderness) of a convex peel were used as image features, and each of the image features was examined for usefulness as an imaging biomarker for NPSLE diagnosis. Here, a 95% convex peel was used.

A specific procedure for generating image features from a brain image data (head MRI image data) in this study is as follows.
(1) One central image is extracted from a set of three-dimensionally-sliced brain images of each case.
(2) Each of the images is a grayscale image, which is converted to a size of 256 × 256.
(3) Skull stripping (removal of the skull from the brain image) is performed.
   This is to eliminate the effects of a significant variation in the luminance of the skull in the brain image depending on the equipment. The present study used a program for deep learning-based skull stripping created by Buda et al. (2019), supra.
(4) The luminance value of the brain image after skull stripping is normalized (the average is subtracted from the luminance value, and the result is divided by the standard deviation), and the minimum value is subtracted from the normalized value to set the minimum value to 0, and then the luminance value of the region other than the brain is set to 0.
(5) For the brain image of each case obtained before this step, a persistence diagram is created using the lower star filtration method, which is a technique suitable for images and lattice data. Here, Dionysus 2 software was used.

### [Results]

A persistence diagram and a 95% convex peel were prepared for each case, and plotted separately for the non-NPSLE group and the NPSLE group. When the perimeters of the 95% convex peel for the black connected components and the white connected components were compared between the non-NPSLE group and the NPSLE group, no significant difference in the perimeter for the black connected components was observed between both groups (provided that P < 0.1), whereas the perimeter for the white connected components is significantly longer in the NPSLE group (FIG. 4 and Table 1).

**[Table 1]**

| Table 1: Comparison of the perimeter (perimeter0) for the black connected components and the perimeter (perimeter1) for the white connected components between the non-NPSLE group and the NPSLE group | | | |
|---|---|---|---|
| | Non-NPSLE | NPSLE | p-value |
| perimeter0, median (IQR) | 7.15 (6.73-7.80) | 7.53 (6.91-8.19) | 0.08 |
| perimeter1, median (IQR) | 6.43 (4.92-7.70) | 7.21 (5.62-8.60) | 0.05 |

| | | | |
|---|---|---|---|
| IQR: interquartile range | | | |

When logistic regression analysis was performed based on the perimeters for the black connected components and the white connected components for NPSLE, the perimeter for the black connected components was not significantly related to NPSLE, and the perimeter for the white connected components was significantly related to NPSLE (Table 2).

**Table 2**

| Table 2: Multiple logistic regression analysis based on the perimeter (perimeter0) for the black connected components and the perimeter (perimeter1) for the white connected components | | | |
|---|---|---|---|
| | Odds ratio | 95% CI | p-value |
| perimeter0 | 1.74 | 0.97 to 3.13 | 0.07 |
| perimeter1 | 1.37 | 1.01 to 1.87 | 0.04 |

| | | | |
|---|---|---|---|
| CI: confidence interval | | | |

When an ROC curve of the perimeter for the white connected components was prepared, AUC = 0.65, which indicates a slightly weak judgment ability (FIG. 5, left). Further, when the cutoff value was searched for by the decision tree method, 27 (90%) out of the 30 cases of NPSLE patients were correctly classified as NPSLE positive when the perimeter for the white connected components was 5.34 or more. However, only 11 (37%) out of the 30 cases of non-NPSLE patients were correctly distinguished as NPSLE negative (FIG. 5, right).

Multiple logistic regression analysis as to whether the patient has NPSLE was performed using a combination of the perimeter for the white connected components and the age, instead of the perimeter alone. This is partly intended for age adjustment because the age is expected to be a confounding factor for white-matter lesions and NPSLE. In this case, both the perimeter for the white connected component and the age were found to exhibit a significant relevance (Table 3).

### Example 3: Acquisition of the indicator of the present invention and construction of the method for acquisition (combination with other indicators)

In this example, the cutoff value was searched for that is used to discriminate NPSLE patients from non-NPSLE patients by additionally taking into account the perimeter for the white connected components and patient characteristics such as blood and cerebrospinal fluid test and oral medicine.

### (1) Discrimination of NPSLE patients according to the age and the perimeter for the white connected components

When multiple logistic regression analysis was performed based on the age and the perimeter for the white connected components to create an ROC curve, NPSLE was discriminated with relatively high performance with AUC = 0.75 (FIG. 6 left and Table 3).

**Table 3.**

| Table 3: Multiple logistic regression analysis based on the age and the perimeter (perimeter1) for the white connected components | | | |
|---|---|---|---|
| | Odds ratio | 95% CI | p-value |
| Age | 0.94 | 0.90 to 0.99 | 0.02 |
| perimeter1 | 1.74 | 1.16 to 2.60 | 0.01 |

| | | | |
|---|---|---|---|
| CI: confidence interval | | | |

Further, when the cutoff value was searched for by the decision tree method, 27 (90%) out of the 30 cases of NPSLE patients were correctly classified as NPSLE positive when the perimeter for the white connected components was 5.34 or more. However, only 11 (37%) out of the 30 cases of non-NPSLE patients were correctly distinguished as NPSLE negative (FIG. 6 right).

When 27 cases of NPSLE patients and 19 cases of non-NPSLE patients, distinguished as NPSLE patients in the first branching, were branched again by the decision tree method, 19 (70%) out of the 27 cases of NPSLE patients were correctly classified as NPSLE positive when the patients were under 44 years of age. In addition, 12 (63%) out of the 19 cases of non-NPSLE patients were correctly distinguished as NPSLE negative (FIG. 6 right).

(2) Discrimination of NPSLE patients with the amount of prednisolone (PSL) orally administered and the perimeter for the white connected components.

When multiple logistic regression analysis was performed based on the amount of PSL orally administered and the perimeter for the white connected component to create an ROC curve, NPSLE patients were discriminated with relatively high performance with AUC = 0.71 (FIG. 7 left and Table 4).

**Table 4**

| Table 4: Multiple logistic regression analysis based on the amount of prednisolone orally administered and the perimeter (perimeter1) for the white connected components | | | |
|---|---|---|---|
| | Odds ratio | 95% CI | p-value |
| Amount of prednisolone orally administered | 1.03 | 0.98 to 1.08 | 0.28 |
| perimeter1 | 1.49 | 1.06 to 2.08 | 0.02 |

| | | | |
|---|---|---|---|
| CI: confidence interval | | | |

Further, when the cutoff value was searched for by the decision tree method, 26 (87%) out of the 30 cases of NPSLE patients were correctly classified as NPSLE positive when the amount of PSL was 4 mg/day or more. However, only 13 cases (43%) of the 30 cases of non-NPSLE patients were correctly distinguished as NPSLE negative.

When 26 cases of NPSLE patients and 17 cases of non-NPSLE patients distinguished as NPSLE patients in the first branching were branched again by the decision tree method, 24 (92%) out of the 26 cases of NPSLE patients were correctly distinguished as NPSLE positive when the perimeter for white connected components was 5.34 or more. In addition, 7 (41%) out of the 17 cases of non-NPSLE patients were correctly distinguished (FIG. 7 right).

### (3) Discrimination of NPSLE patients based on the amount of C3 in serum and the perimeter for the white connected components

When multiple logistic regression analysis was performed based on the amount of C3 in serum (mg/dL) and the perimeter for the white connected components to create an ROC curve, NPSLE was discriminated with relatively high performance with AUC = 0.73 (FIG. 8 left and Table 5).

**Table 5**

| Table 5: Multiple logistic regression analysis based on C3 in serum and the perimeter (perimeter1) for the white connected components | | | |
|---|---|---|---|
| | Odds ratio | 95% CI | p-value |
| C3 in serum | 1.03 | 1.00 to 1.05 | 0.03 |
| perimeter1 | 1.46 | 1.04 to 2.04 | 0.03 |

| | | | |
|---|---|---|---|
| CI: confidence interval | | | |

Further, when the cutoff value was searched for by the decision tree method, 27 (90%) out of the 30 cases of NPSLE patients were correctly classified as NPSLE positive when the perimeter for the white connected components was 5.34 or more. However, only 11 (37%) out of the 30 cases of non-NPSLE patients were correctly distinguished as NPSLE negative.

When 27 NPSLE patients and 19 non-NPSLE patients distinguished as NPSLE patients in the first branching were branched again by the decision tree method, 7 (26%) out of 27 NPSLE patients were correctly classified as NPSLE positive when the concentration of C3 in serum was 91. 4 mg/dL or more. In addition, 19 (100%) out of 19 cases of non-NPSLE patients were correctly distinguished as NPSLE negative (FIG. 8 right).

### (4) Discrimination of NPSLE patients based on the blood glucose level and perimeter for the white connected components

When multiple logistic regression analysis was performed based on the blood glucose level (mg/dL) and the perimeter for the white connected components to create an ROC curve, NPSLE was discriminated with relatively high performance with AUC = 0.73 (FIG. 9 left and Table 6).

**Table 6**

| Table 6: Multiple logistic regression analysis based on the blood glucose level and the perimeter (perimeter1) for the white connected components | | | |
|---|---|---|---|
| | Odds ratio | 95% CI | p-value |
| Blood glucose level | 0.99 | 0.96 to 1.01 | 0.46 |
| perimeter1 | 1.62 | 1.13 to 2.55 | 0.02 |

| | | | |
|---|---|---|---|
| CI: confidence interval | | | |

Further, when the cutoff value was searched for by the decision tree method, 27 (90%) out of the 30 cases of NPSLE patients were correctly classified as NPSLE positive when the perimeter for the white connected components was 5.34 or more. However, only 11 (37%) out of the 30 cases of non-NPSLE patients were correctly distinguished as NPSLE negative.

When 3 cases of NPSLE patients and 11 cases of non-NPSLE patients distinguished as non-NPSLE patients in the first branching were branched again by the decision tree method, 3 (100%) out of the 3 NPSLE patients were correctly classified as NPSLE positive when the blood glucose level was 92 mg/dL or more. In addition, 8 out of the 11 cases (73%) out of non-NPSLE patients were correctly distinguished as NPSLE negative (FIG. 9 right).

### (5) Discrimination of NPSLE patients based on the amount of IL-6 in the cerebrospinal fluid and the perimeter for the white connected components

When multiple logistic regression analysis was performed based on IL-6 in the cerebrospinal fluid (pg/mL) and the perimeter for the white connected components to create an ROC curve, NPSLE was discriminated with relatively high performance with AUC = 0.74 (FIG. 10 left and Table 7).

**Table 7**

| Table 7: Multiple logistic regression analysis based on IL-6 in the cerebrospinal fluid and the perimeter (perimeter1) for the white connected components | | | |
|---|---|---|---|
| | Odds ratio | 95% CI | p-value |
| IL-6 in the cerebrospinal fluid | 1.07 | 1.00 to 1.25 | 0.23 |
| perimeter1 | 1.40 | 0.93 to 2.19 | 0.12 |

| | | | |
|---|---|---|---|
| CI: confidence interval | | | |

Further, when the cutoff value was searched for by the decision tree method, 7 cases (23%) of the 30 cases of NPSLE patients were correctly classified as NPSLE positive when the concentration of IL-6 in the cerebrospinal fluid was 22.8 pg/mL or more. Additionally, 24 (80%) out of the 30 cases of non-NPSLE patients were correctly distinguished as NPSLE negative.

When 23 cases of NPSLE patients and 24 cases of non-NPSLE patients distinguished as non-NPSLE patients in the first branching were branched again by the decision tree method, 20 (87%) out of the 23 cases of NPSLE patients were correctly classified as NPSLE positive when the perimeter for the white connected components was 5.60 or more. In addition, 10 (42%) out of 24 cases of non-NPSLE patients were correctly distinguished as NPSLE negative (FIG. 10 right).

### Example 4: Acquisition of the indicator of the present invention and construction of the method for acquisition (each analysis) (II)

For the image features from the brain image (head MRI image data) in the present study, a method similar to that in Examples 1 and 2 was used to search for effective image features other than the perimeter (perimeter1) of the 95% convex peel for the white connected components obtained in Example 2.

### [Results]

As in Example 2, a persistence diagram and a 95% convex peel were prepared for each case and plotted separately for the non-NPSLE group and the NPSLE group. The area (area1) of the 95% convex peel for the white connected components was compared between the non-NPSLE group and the NPSLE group. As a result, the area of the white connected components was significantly larger in the NPSLE group (FIG. 11 and Table 8).

**Table 8.**

| Table 8: Comparison of the area (area1) of the white connected components between the non-NPSLE group and the NPSLE group | | | |
|---|---|---|---|
| | Non-NPSLE | NPSLE | p-value |
| area1, median (IQR) | 1.07 (0.75 to 1.39) | 1.32 (0.99 to 1.86) | 0.03 |

| | | | |
|---|---|---|---|
| IQR: interquartile range | | | |

Additionally, when logistic regression analysis was performed based on the area (area1) of the white connected components for NPSLE, the area of the white connected components was significantly associated with NPSLE (Table 9).

**Table 9.**

| Table 9: Multiple logistic regression analysis based on the area (area1) of the white connected components | | | |
|---|---|---|---|
| | Odds ratio | 95% CI | p-value |
| area1 | 2.67 | 1.04 to 6.87 | 0.04 |

| | | | |
|---|---|---|---|
| CI: confidence interval | | | |

When an ROC curve of the area of the white connected components was prepared, the judgment ability was slightly weak with AUC = 0.66 (FIG. 12 left). When the cutoff value was further searched for by the decision tree method, 30 (100%) out of the 30 cases of NPSLE patients were correctly classified as NPSLE positive when the area of the white connected components was 0.597 or more. However, only 5 (17%) out of the 30 cases of non-NPSLE patients were correctly distinguished as NPSLE negative (FIG. 12 right).

### Example 5: Acquisition of the indicator of the present invention and construction of the method for acquisition (combination with other indicators) (II)

In the present example, a technique similar to that in Example 3 was used to search for a cutoff value for discriminating NPSLE patients from non-NPSLE patients by additionally taking into account patient characteristics such as the area of the white connected components, the age, the history of cerebrovascular disorder, the serum complement value (CH50), C3 in serum, C4 in serum, the oral medicine (PSL), the number of platelets, the amount of serum anti-SSB antibodies, and lupus anticoagulant.

### (1) Discrimination of NPSLE patients according to the age, the history of cerebrovascular disorder, the serum complement value (CH50), and the area of the white connected components

When multiple logistic regression analysis was performed based on the age, the history of cerebrovascular disorder, the serum complement value (CH50), and the area of the white connected components to create an ROC curve, NPSLE was discriminated with relatively high performance with AUC = 0.82 (FIG. 13 and Table 10).

**[Table 10]**

| Table 10: Multiple logistic regression analysis based on the age, the presence or absence of a cerebrovascular disease, CH50, and the area (area1) of the white connected components. | | | |
|---|---|---|---|
| | Odds ratio | 95% CI | p-value |
| Age | 0.93 | 0.87 to 0.98 | 0.02 |
| Presence or absence of a cerebrovascular disease | 9.74 | 1.61 to 93.0 | 0.02 |
| CH50 | 1.07 | 1.01 to 1.15 | 0.04 |
| area1 | 4.42 | 1.35 to 19.6 | 0.03 |

| | | | |
|---|---|---|---|
| CI: confidence interval | | | |

FIG. 14 illustrates the result of a search for the cutoff value by the decision tree method. When the cutoff value was searched for by the decision tree method, 9 (30%) out of the 30 cases of NPSLE patients were correctly classified as NPSLE positive when the patients previously had a cerebrovascular disease. 27 (90%) out of the 30 non-NPSLE patients were correctly distinguished as NPSLE negative (FIG. 14). When 9 cases of NPSLE patients and 3 cases of non-NPSLE patients distinguished as NPSLE patients in the first branching were branched again by the decision tree method, 5 (56%) out of the 9 NPSLE patients were correctly classified as NPSLE positive when the patients were less than 48 years of age. Additionally, 3 (100%) out of the 3 non-NPSLE patients were correctly distinguished as NPSLE negative (FIG. 14).

When 21 cases of NPSLE patients and 27 cases of non-NPSLE patients distinguished as non-NPSLE patients in the first branching were branched again by the decision tree method, 19 (90%) out of the 21 cases of NPSLE patients were correctly classified as NPSLE positive when the concentration of CH50 was 24 U/mL or more. Additionally, 13 (48%) out of the 27 non-NPSLE patients were correctly distinguished as NPSLE negative (FIG. 14). In addition, when 19 cases of NPSLE patients and 14 cases of non-NPSLE patients distinguished as NPSLE patients in the second branching were branched again by the decision tree method, 17 (89%)out of the 19 NPSLE patients were correctly classified as NPSLE positive when the patients were less than 53 years of age. Additionally, 6 (43%) out of the 14 non-NPSLE patients were correctly distinguished as NPSLE negative (FIG. 14). Further, when 17 cases of NPSLE patients and 8 non-NPSLE patients distinguished as NPSLE patients in the third branching were branched again by the decision tree method, 15 (88%) out of the 17 NPSLE patients were correctly classified as NPSLE positive when the area (area1) of the white connected components was 0.802 or more. 4 (50%) of the 8 non-NPSLE patients were correctly distinguished as NPSLE negative (FIG. 14).

### (2) Discrimination of NPSLE patients based on the serum complement value (CH50) and the area of the white connected components

When multiple logistic regression analysis was performed based on the serum complement value (CH50) and the area of the white connected components to create an ROC curve, NPSLE was discriminated with relatively high performance with AUC = 0.72 (FIG. 15 left and Table 11).

**[Table 11]**

| Table 11: Multiple logistic regression analysis based on CH50 and the area (area1) of the white connected components | | | |
|---|---|---|---|
| | Odds ratio | 95% CI | p-value |
| CH50 | 1.07 | 1.01 to 1.13 | 0.03 |
| area1 | 3.01 | 1.07 to 8.49 | 0.04 |

| | | | |
|---|---|---|---|
| CI: confidence interval | | | |

FIG. 15 right illustrates the result of a search for the cutoff value by the decision tree method. When the cutoff value was searched for by the decision tree method, 30 (100%) out of the 30 cases of NPSLE patients were correctly classified as NPSLE positive when the area of the white connected components was 0.597 or more. However, only 5 (17%) out of the 30 cases of non-NPSLE patients were correctly distinguished as NPSLE negative (FIG. 15 right).

When 30 cases of NPSLE patients and 25 cases of non-NPSLE patients distinguished as NPSLE patients in the first branching were branched again by the decision tree method, 28 (93%) out of the 30 cases of NPSLE patients were correctly classified as NPSLE positive when the concentration of CH50 was 20.7 U/mL or more. In addition, 8 out of the 25 (32%) non-NPSLE patients were correctly distinguished as NPSLE negative (FIG. 15 right).

### (3) Discrimination of NPSLE patients according to the age and the area of the white connected components

When multiple logistic regression analysis was performed based on the age and the area of the white connected components to create an ROC curve, NPSLE was discriminated with relatively high performance with AUC = 0.73 (FIG. 16 left and Table 12).

**[Table 12]**

| Table 12: Multiple logistic regression analysis based on the age and the area (area1) of the white connected components | | | |
|---|---|---|---|
| | Odds ratio | 95% CI | p-value |
| Age | 0.95 | 0.91 to 1.00 | 0.03 |
| area1 | 4.40 | 1.39 to 13.9 | 0.01 |

| | | | |
|---|---|---|---|
| CI: confidence interval | | | |

FIG. 16 right illustrates the result of a search for the cutoff value by the decision tree method. When the cutoff value was further searched for by the decision tree method, 30 (100%) out of the 30 cases of NPSLE patients were correctly classified as NPSLE positive when the area of the white connected components was 0.597 or more. However, only 5 (17%) out of 30 cases of non-NPSLE patients were correctly distinguished as NPSLE negative (FIG. 16, right).

When 30 cases of NPSLE patients and 25 cases of non-NPSLE patients distinguished as NPSLE patients in the first branching were branched again by the decision tree method, 21 (70%) out of the 30 cases of NPSLE patients were correctly classified as NPSLE positive when the patients were less than 44 years of age. In addition, 15 (60%) out of the 25 cases of non-NPSLE patients were correctly distinguished as NPSLE negative (FIG. 16 right).

### (4) Discrimination of NPSLE patients based on the amount of C3 in serum and the area of white connected components

When multiple logistic regression analysis was performed based on the amount of C3 in serum and the area of the white connected components to create an ROC curve. NPSLE was discriminated with relatively high performance with AUC = 0.73 (FIG. 17 left and Table 13).

**[Table 13]**

| Table 13: Multiple logistic regression analysis based on C3 and the area (area1) of the white connected components | | | |
|---|---|---|---|
| | Odds ratio | 95% CI | p-value |
| C3 | 1.03 | 1.00 to 1.05 | 0.04 |
| area1 | 3.04 | 1.09 to 8.45 | 0.03 |

| | | | |
|---|---|---|---|
| CI: confidence interval | | | |

FIG. 17 right illustrates the result of a search for the cutoff value by the decision tree method. When the cutoff value was further searched for by the decision tree method, 30 (100%) out of the 30 cases of NPSLE patients were correctly classified as NPSLE positive when the area of the white connected components was 0.597 or more. However, only 5 (17%) out of 30 cases of non-NPSLE patients were correctly distinguished as NPSLE negative (FIG. 17 right).

When 30 cases of NPSLE patients and 25 cases of non-NPSLE patients distinguished as NPSLE patients in the first branching were branched again by the decision tree method, 18 (60%) out of the 30 cases of NPSLE patients were correctly classified as NPSLE positive when the concentration of C3 in serum was 67.7 mg/dL or more. In addition, 18 (72%) out of the 25 non-NPSLE patients were correctly distinguished as NPSLE negative (FIG. 17 right).

### (5) Discrimination of NPSLE patients based on the amount of C4 in serum and the area of the white connected components

When multiple logistic regression analysis was performed based on the amount of C4 in serum and the area of the white connected components to create an ROC curve, NPSLE was discriminated with relatively high performance with AUC = 0.74 (FIG. 18 left and Table 14).

**[Table 14]**

| Table 14: Multiple logistic regression analysis based on the age and the area (area1) of the white connected components | | | |
|---|---|---|---|
| | Odds ratio | 95% CI | p-value |
| C4 | 1.07 | 0.99 to 1.16 | 0.07 |
| Area1 | 2.29 | 0.82 to 6.35 | 0.11 |

| | | | |
|---|---|---|---|
| CI: confidence interval | | | |

FIG. 18 right illustrates the result of a search for the cutoff value by the decision tree method. Further, when the cutoff value was searched for by the decision tree method, 23 (77%) out of the 30 cases of NPSLE patients were correctly classified as NPSLE positive when the concentration of C4 in serum was 9.6 mg/dL or more. However, only 17 (57%) out of the 30 cases of non-NPSLE patients were correctly distinguished as NPSLE negative (FIG. 18 right).

When 7 cases of NPSLE patients and 17 cases of non-NPSLE patients distinguished as non-NPSLE patients in the first branching were branched again by the decision tree method, 7 (100%) out of the 7 cases of NPSLE patients were correctly classified as NPSLE positive when the area of the white connected components was 1.12 or more. In addition, 11 (65%) out of the 17 cases of non-NPSLE patients were correctly distinguished as NPSLE negative (FIG. 18 right).

### (6) Discrimination of NPSLE patients based on the amount of prednisolone (PSL) orally administered and the area of the white connected components

When multiple logistic regression analysis was performed based on the amount of prednisolone (PSL) orally administered and the area of the white connected components to create an ROC curve, NPSLE was discriminated with relatively high performance with AUC = 0.72 (FIG. 19 left and Table 15).

**[Table 15]**

| Table 15: Multiple logistic regression analysis based on the amount of PSL orally administered and the area (area1) of the white connected components | | | |
|---|---|---|---|
| | Odds ratio | 95% CI | p-value |
| PSL | 1.02 | 0.98 to 1.07 | 0.35 |
| area1 | 3.20 | 1.16 to 8.88 | 0.03 |
| CI: confidence interval | | | |

FIG. 19 right illustrates the result of a search for the cutoff value by the decision tree method. FIG. 19 right illustrates the result of a search for the cutoff value by the decision tree method. Further, when the cutoff value was searched for by the decision tree method, 26 (87%) out of the 30 cases of NPSLE patients were correctly classified as NPSLE positive when the amount of PSL was 4 mg/day or more. However, only 14 (47%) out of the 30 cases of non-NPSLE patients were correctly distinguished as NPSLE negative (FIG. 19 right).

When 26 cases of NPSLE patients and 16 cases of non-NPSLE patients distinguished as NPSLE patients in the first branching were branched again by the decision tree method, 23 (88%) out of the 26 cases of NPSLE patients were correctly classified as NPSLE positive when the area of the white connected components was 0.96 or more. In addition, 8 (50%) out of the 16 cases of non-NPSLE patients were correctly distinguished as NPSLE negative (FIG. 19 right).

### (7) Discrimination of NPSLE patients based on the presence or absence of renal disorder and the area of the white connected components

When multiple logistic regression analysis was performed based on the presence or absence of renal disorder and the area of the white connected components to create an ROC curve, NPSLE was discriminated with relatively high performance with AUC = 0.72 (FIG. 20 left and Table 16).

**[Table 16]**

| Table 16: Multiple logistic regression analysis based on the age and the area (area1) of the white connected components | | | |
|---|---|---|---|
| | Odds ratio | 95% CI | p-value |
| Presence or absence of renal disorder | 0.46 | 0.15 to 1.39 | 0.17 |
| area1 | 2.80 | 1.05 to 7.46 | 0.04 |
| CI: confidence interval | | | |

FIG. 20 right illustrates the result of a search for the cutoff value by the decision tree method. When the cutoff value was further searched for by the decision tree method, 30 (100%) out of the 30 cases of NPSLE patients were correctly classified as NPSLE positive when the area of the white connected components was 0.597 or more. However, only 5 (17%) out of the 30 cases of non-NPSLE patients were correctly distinguished as NPSLE negative (FIG. 20 right).

When 30 cases of NPSLE patients and 25 cases of non-NPSLE patients distinguished as NPSLE patients in the first branching were branched again by the decision tree method, 21 (70%) out of the 30 cases of NPSLE patients were correctly classified as NPSLE positive when the patients had no renal disorder. In addition, 12 (48%) out of the 25 non-NPSLE patients were correctly distinguished as NPSLE negative (FIG. 20 right).

### (8) Discrimination of NPSLE patients based on the number of platelets and the area of the white connected components

When multiple logistic regression analysis was performed based on the number of platelets and the area of the white connected components to create an ROC curve, NPSLE was discriminated with relatively high performance with AUC = 0.70 (FIG. 21 left and Table 17).

**[Table 17]**

| Table 17: Multiple logistic regression analysis based on the number of platelets and the area (area1) of the white connected components | | | |
|---|---|---|---|
| | Odds ratio | 95% CI | p-value |
| Number of platelets | 1.04 | 0.97 to 1.11 | 0.27 |
| area1 | 2.86 | 1.09 to 7.53 | 0.03 |

| | | | |
|---|---|---|---|
| CI: confidence interval | | | |

FIG. 21 right illustrates the result of a search for the cutoff value by the decision tree method. When the cutoff value was searched for by the decision tree method, 30 (100%) out of the 30 cases of NPSLE patients were correctly classified as NPSLE positive when the area of the white connected components was 0.597 or more. However, only 5 (17%) out of 30 cases of non-NPSLE patients were correctly distinguished as NPSLE negative (FIG. 21 right).

When 30 cases of NPSLE patients and 25 cases of non-NPSLE patients distinguished as NPSLE patients in the first branching were branched again by the decision tree method, 8 (27%) out of the 30 cases of NPSLE patients were correctly classified as NPSLE positive when the area of the white connected components was 1.77 or more. In addition, 23 (92%) out of the 25 cases of non-NPSLE patients were correctly distinguished as NPSLE negative (FIG. 21 right).

### (9) Discrimination of NPSLE patients based on the amount of serum anti-SSB antibodies and the area of the white connected components

When multiple logistic regression analysis was performed based on the number of platelets and the area of the white connected components to create an ROC curve, NPSLE was discriminated with relatively high performance with AUC = 0.74 (FIG. 22 left and Table 18).

**[Table 18]**

| Table 18: Multiple logistic regression analysis based on the age and the area (area1) of the white connected components | | | |
|---|---|---|---|
| | Odds ratio | 95% CI | p-value |
| Anti-SSB antibodies | 0.99 | 0.98 to 1.01 | 0.31 |
| area1 | 3.24 | 1.13 to 9.29 | 0.03 |

| | | | |
|---|---|---|---|
| CI: confidence interval | | | |

FIG. 22 right illustrates the result of a search for the cutoff value by the decision tree method. FIG. 22 right illustrates the result of a search for the cutoff value by the decision tree method. When the cutoff value was searched for by the decision tree method, 19 (63%) out of the 30 cases of NPSLE patients were correctly classified as NPSLE positive when the amount of anti-SSB antibodies was less than 2.2 U/mL. Additionally, 22 (73%) out of the 30 cases of non-NPSLE patients were correctly distinguished as NPSLE negative (FIG. 22 right).

When 19 cases of NPSLE patients and 8 cases of non-NPSLE patients distinguished as NPSLE patients in the first branching were branched again by the decision tree method, 15 (79%) out of the 19 cases of NPSLE patients were correctly classified as NPSLE positive when the area of the white connected components was 1.06 or more. In addition, 5 (63%) out of the 8 cases of non-NPSLE patients were correctly distinguished as NPSLE negative (FIG. 22 right).

### (10) Discrimination of NPSLE patients based on lupus anticoagulant and the area of the white connected components

When multiple logistic regression analysis was performed based on lupus anticoagulant and the area of the white connected components to create an ROC curve, NPSLE was discriminated with relatively high performance with AUC = 0.73 (FIG. 23 left and Table 19).

**[Table 19]**

| Table 19: Multiple logistic regression analysis based on the age and the area (area1) of the white connected components | | | |
|---|---|---|---|
| | Odds ratio | 95% CI | p-value |
| Serum lupus anticoagulant | 0.25 | 0.03 to 2.23 | 0.21 |
| area1 | 6.86 | 1.80 to 26.1 | 0.005 |

| | | | |
|---|---|---|---|
| CI: confidence interval | | | |

FIG. 23 right illustrates the result of a search for the cutoff value by the decision tree method. When the cutoff value was further searched for by the decision tree method, 30 (100%) out of the 30 cases of NPSLE patients were correctly classified as NPSLE positive when the area of the white connected components was 0.597 or more. However, only 5 (17%) out of 30 cases of non-NPSLE patients were correctly distinguished as NPSLE negative (FIG. 23, right).

When 30 cases of NPSLE patients and 25 cases of non-NPSLE patients distinguished as NPSLE patients in the first branching were branched again by the decision tree method, 8 (27%) out of the 30 cases of NPSLE patients were correctly classified as NPSLE positive when the area of the white connected components was 1.77 or more. In addition, 23 (92%) out of the 25 cases of non-NPSLE patients were correctly distinguished as NPSLE negative (FIG. 23 right).

### Example 6: Acquisition of the indicator of the present invention and construction of the method for acquisition (combination with other indicators) (III)

In this example, based on the result of (1) of Example 5, a technique similar to that in Example 3 was used to discriminate NPSLE patients by using the perimeter (perimeter1) of the white connected components, the age, the history of cerebrovascular disorder, and the serum complement value (CH50).

When multiple logistic regression analysis was performed based on the age, the history of cerebrovascular disorder, the serum complement value (CH50), and the perimeter for the white connected components to create an ROC curve, NPSLE was discriminated with relatively high performance with AUC = 0.83 (FIG. 24 and Table 20).

**[Table 20]**

| Table 20: Multiple logistic regression analysis based on the age, the presence or absence of a cerebrovascular disease, CH50, the perimeter (perimeter1) of the white connected components. | | | |
|---|---|---|---|
| | Odds ratio | 95% CI | p-value |
| Age | 0.93 | 0.88 to 0.99 | 0.01 |
| Presence or absence of a cerebrovascular disease | 8.46 | 1.24 to 57.5 | 0.03 |
| CH50 | 1.07 | 1.00 to 1.14 | 0.04 |
| perimeter1 | 1.67 | 1.07 to 2.63 | 0.03 |

| | | | |
|---|---|---|---|
| CI: confidence interval | | | |

FIG. 25 illustrates the result of a search for the cutoff value by the decision tree method. When the cutoff value was searched for by the decision tree method, 9 (30%) out of the 30 cases of NPSLE patients were correctly classified as NPSLE positive when the patients previously had a cerebrovascular disease. 27 (90%) of the 30 non-NPSLE patients were correctly distinguished as NPSLE negative (FIG. 25).

When 21 cases of NPSLE patients and 27 cases of non-NPSLE patients distinguished as non-NPSLE patients in the first branching were branched again by the decision tree method, 19 (90%) out of the 21 cases of NPSLE patients were correctly classified as NPSLE positive when the concentration of CH50 was 24 U/mL or more. Additionally, 13 (48%) out of the 27 non-NPSLE patients were correctly distinguished as NPSLE negative (FIG. 25).

In addition, when two cases of NPSLE patients and 13 cases of non-NPSLE patients distinguished as non-NPSLE patients in the second branching were branched again by the decision tree method, two (100%) out of the two cases of NPSLE patients were correctly classified as NPSLE positive when the perimeter (perimeter1) of the white connected components is 7.00 or more. 10 of the 13 (77%) non-NPSLE patients were correctly distinguished as NPSLE negative (FIG. 25).

The above results suggest that the method of the present invention including the step of acquiring an image feature from a persistence diagram created using a brain image is suitable and useful for acquiring an indicator for diagnosing whether a subject is an SLE patient or an NPSLE patient. Additionally, in particular, it can be understood that the white connected components obtained in the present study are excellent as an imaging biomarker for discriminating NPSLE from non-NPSLE (SLE from NPSLE). In addition, the white connected components, corresponding to the imaging biomarker obtained in this study, are sufficiently excellent for discriminating between NPSLE and non-NPSLE (between SLE and NPSLE), but a combination of the imaging biomarker with any of the patient characteristics (in particular, the age of the subject, the amount of prednisolone orally administered to the subject, the amount of C3 in serum, the blood glucose level, the amount of IL-6 in the cerebrospinal fluid, the presence or absence of a previous cerebrovascular disease, the serum complement value CH50, the amount of C4 in serum, the presence or absence of renal disorder, the number of platelets, the amount of serum anti-SSB antibodies, and serum lupus anticoagulant) exhibits an AUC, which is a statistic, of 0.7 or more, suggesting that the combination of the imaging biomarker with any of the characteristics is very suitable and useful as a model for discriminating NPSLE from non-NPSLE (SLE from NPSLE).

### Industrial Applicability

According to the present invention, for NPSLE, for which definitive diagnosis is difficult, imaging biomarkers (indicators) can be obtained that are used to diagnose whether a subject (patient) is an SLE patient or an NPSLE patient (whether the subject is NPSLE positive or negative). In addition, using the imaging biomarker obtained by the present invention allows an NPSLE group to be distinguished from an SLE group with NPSLE non-progressing based on head MRI images. Then, the distinction result can be used to assist a physician in a definitive diagnosis, and can further contribute to appropriate drug intervention for NPSLE or SLE. Thus, the distinction result is particularly useful in the medical field or the like.

This application is based on a patent application JP 2022-023098 filed in Japan (filing date: Feb. 17, 2021), the contents of which are incorporated in full herein by reference.

## Claims

1. A method for obtaining an indicator for diagnosing whether a subject is an SLE patient or an NPSLE patient, the method comprising:
(1) preparing head MRI images of SLE patients and NPSLE patients;
(2) gradually changing a color tone of each of the head MRI images;
(3) detecting and counting white connected components and black connected components generated in response to color tone changes;
(4) obtaining a scatter diagram of times of generation and disappearance of the white connected components and the black connected components based on a result of the detecting and counting (3);
(5) verifying whether there is a significant difference in any of image features between the SLE patients and the NPSLE patients, the image features being obtained from the scatter diagram; and
(6) identifying, as the indicator, an image feature in which a significant difference is recognized.

2. The method according to claim 1, wherein
the preparing (1) includes pre-processing the head MRI image.

3. The method according to claim 1 or 2, wherein
in the verifying (5), the image features are obtained based on a convex peel containing at least 90% of plots in the scatter diagram.

4. The method according to claim 3, wherein
the image features are obtained based on a length of a perimeter of the convex peel containing at least 90% of the plots in the scatter diagram.

5. A method for assisting diagnosis of whether a subject is an SLE patient or an NPSLE patient, the method comprising:
(1) preparing a head MRI image of a subject;
(2) gradually changing a color tone of the head MRI image;
(3) detecting and counting white connected components or black connected components generated in response to color tone changes;
(4) obtaining a scatter diagram of times of generation and disappearance of the white connected components and the black connected components based on a result of the detecting and counting (3);
(5) calculating a specific image feature from the scatter diagram; and
(6) determining whether the subject is an SLE patient or an NPSLE patient from the calculated image feature.

6. The method according to claim 5, wherein
the preparing (1) includes pre-processing the head MRI image.

7. The method according to claim 5 or 6, wherein
in the calculating (5), the specific image feature is a length of a perimeter of a convex peel containing at least 90% of plots in the scatter diagram.

8. The method according to claim 7, wherein
in the determining (6), whether the subject is an SLE patient or an NPSLE patient is determined by comparing the length of the perimeter calculated in the calculating (5) with a predetermined cutoff value.

9. The method according to claim 5 or 6, wherein
in the calculating (5), the specific image feature is an area of a convex peel within which at least 90% of plots in the scatter diagram.

10. The method according to claim 9, wherein
in the determining (6), whether the subject is an SLE patient or an NPSLE patient is determined by comparing the area calculated in the calculating (5) with a predetermined cutoff value.

11. The method according to any one of claims 5 to 7 and 9, wherein
in the determining (6), whether the subject is an SLE patient or an NPSLE patients is determined from:
the calculated image feature; and
one or more indicators selected from the group consisting of an age of the subject, an amount of prednisolone orally administered, an amount of C3 in serum, a blood glucose level, and an amount of IL-6 in a cerebrospinal fluid.

12. A computer program for predicting whether a subject is an SLE patient or an NPSLE patient, the computer program causing a computer to function as:
a reception unit (P1) configured to receive head MRI image data of a prediction target;
a calculation unit (P2) configured to
(i) gradually change a color tone of the received head MRI image data,
(ii) detect and count white connected components or black connected components generated in response to color tone changes,
(iii) obtain a scatter diagram of times of generation and disappearance of the white connected components and the black connected components based on a result of the detecting and counting,
(iv) calculate a specific image feature from the scatter diagram, and
(v) calculate at least one of a probability that the subject is an SLE patient or a probability that the subject is an NPSLE patient; and
an output unit (P3) configured to output a calculation result obtained by the calculation unit.
